(19) Europäisches Patentamt
European Patent Office
Office européen des brevets

(11) Publication number: **0 270 376 B1**

(12) **EUROPEAN PATENT SPECIFICATION**

(45) Date of publication of patent specification: **30.09.92**

(51) Int. Cl.5: **C07K 7/10**, A61K 37/24, C07K 1/00

(21) Application number: **87310695.9**

(22) Date of filing: **04.12.87**

(54) **Calcitonin gene-related peptide derivatives.**

(30) Priority: **04.12.86 JP 289597/86**
**25.12.86 JP 310585/86**

(43) Date of publication of application:
**08.06.88 Bulletin 88/23**

(45) Publication of the grant of the patent:
**30.09.92 Bulletin 92/40**

(84) Designated Contracting States:
**DE ES FR GB IT**

(56) References cited:
**WO-A-85/00165**

**CHEM. PHARM. BULL., vol. 34, no. 9, September 1986, pages 3915-3918, Tokyo, JP; A. OTAKA et al.: "Studies on pepptides. CXLII. 1.2) Synthesis of des-I-Ala-des-alpha-amino-human calcitonin gene-related peptide"**

**FEBS LETTERS, vol. 203, no. 1, July 1986, pages 7-10, Elsevier Science Publishers B.V. (Biomedical Division), Amsterdam, NL; S. MINVIELLE et al.: "Isolation and partial characterization of the calcitonin gene in a lower vertebrate"**

(73) Proprietor: **TOYO JOZO KABUSHIKI KAISHA**
**632-1 Mifuku Ohito-cho**
**Tagata-gun Shizuoka-ken(JP)**

(72) Inventor: **Morita, Kaoru**
**486-4, Kashiwakubo Shuzenji-cho**
**Tagata-gun Shizuoka-ken(JP)**
Inventor: **Uzawa, Toyonobu**
**632-1, Mifuku Ohito-cho**
**Tagata-gun Shizuoka-ken(JP)**
Inventor: **Hori, Masayuki**
**1588-1, Nagoya Nirayama-cho**
**Tagata-gun Shizuoka-ken(JP)**
Inventor: **Noda, Toshiharu**
**908-5, Osaka Izu-Nagaoka-cho**
**Tagata-gun Shizuoka-ken(JP)**

(74) Representative: **Woods, Geoffrey Corlett et al**
**J.A. KEMP & CO. 14 South Square Gray's Inn**
**London WC1R 5EU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to calcitonin gene-related peptide derivatives, to their preparation and to pharmaceutical compositions containing them.

Recently, the amino acid sequence of chicken calcitonin gene-related peptide (c-CGRP) has been elucidated by analysis of the DNA sequence of chicken [FEBS Letters, vol. 203, No. 1, 7-10 July, 1986]. However, there has been no report of the isolation and synthesis of c-CGRP. The activity and action of c-CGRP are therefore not known.

We have synthesized derivatives of c-CGRP and have made comparative studies of their biological activities. We have found that they have a stronger serum calcium reducing activity and serum phosphate reducing activity with superior prolonged action as compared with human CGRP (h-CGRP as disclosed in Nature, 308 (19):746-748 (1984); Neuropeptides, 4: 425-434 (1985); and Nature, 313 (3): 54-56 (1984)).

Accordingly the present invention provides c-CGRP derivatives of the formula (1):

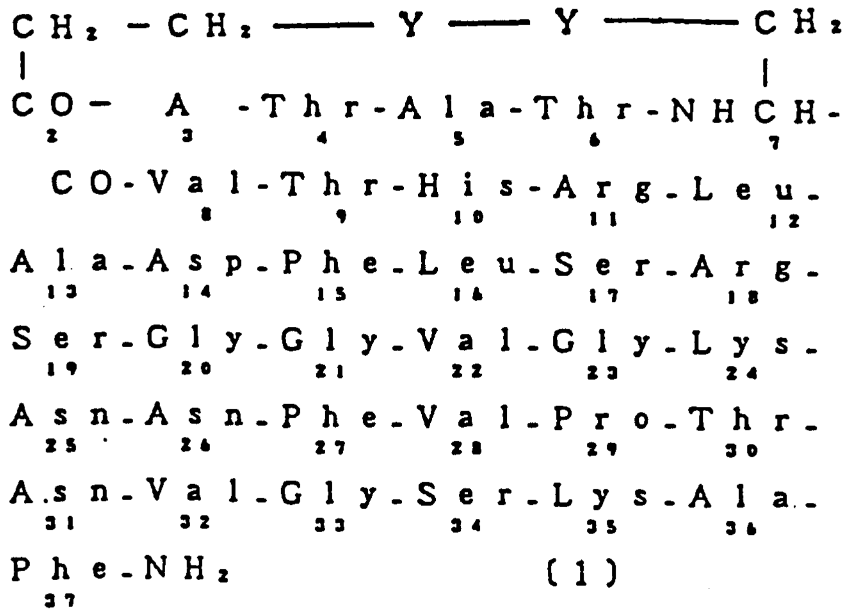

(1)

wherein Y is sulfur or methylene and A is Asn or Asp, or a physiologically acceptable salt thereof.

In the present invention, a peptide (1) wherein Y is sulfur and A is Asn is designated as desalanyl-deamino-c-CGRP. A peptide (1) wherein Y is sulfur and A is Asp is designated as desalanyl-deamino-[$Asp^3$]-c-CGRP. A peptide (1) wherein Y is methylene and A is Asn is designated as desalanyl-[$Asu^{2,7}$]-c-CGRP. A peptide (1) wherein Y is methylene and A is Asp is designated as desalanyl-[$Asp^3$, $Asu^{2,7}$]-c-CGRP. The salts are typically physiologically acceptable salts.

The invention also provides pharmaceutical compositions comprising a peptide of formula (1) or a physiologically acceptable salt thereof as active ingredient and a pharmaceutically acceptable carrier or diluent.

The drawings of this specification illustrate the following:

| | |
|---|---|
| Fig. 1: | Rat serum calcium reducing effect of h-CGRP, c-CGRP and desalanyl-deamino-c-CGRP of the present invention; |
| Fig. 2: | Rat serum inorganic phosphate reducing effect of h-CGRP, c-CGRP and desalanyl-deamino-c-CGRP; |
| Fig. 3: | Rat serum calcium reducing effect of h-CGRP, c-CGRP, desalanyl-[$Asu^{2,7}$]-c-CGRP and desalanyl-[$Asp^3$, $Asu^{2,7}$]-c-CGRP of the present invention; |
| Fig. 4: | Rat serum inorganic phosphate reducing effect of h-CGRP, c-CGRP, desalanyl-[$Asu^{2,7}$]-c-CGRP and desalanyl-[$Asp^3$, $Asu^{2,7}$]-c-CGRP of the present invention; |
| Fig. 5 and Fig. 6: | Process chart for synthesis of intermediate peptide fragment (3-8). |

Preferred compounds are peptides of the following three formulae or physiologically acceptable salts thereof:

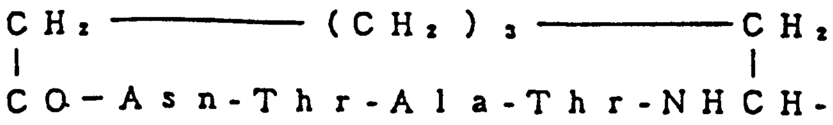

CO-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-Val-Gly-Ser-Lys-Ala-Phe-$NH_2$

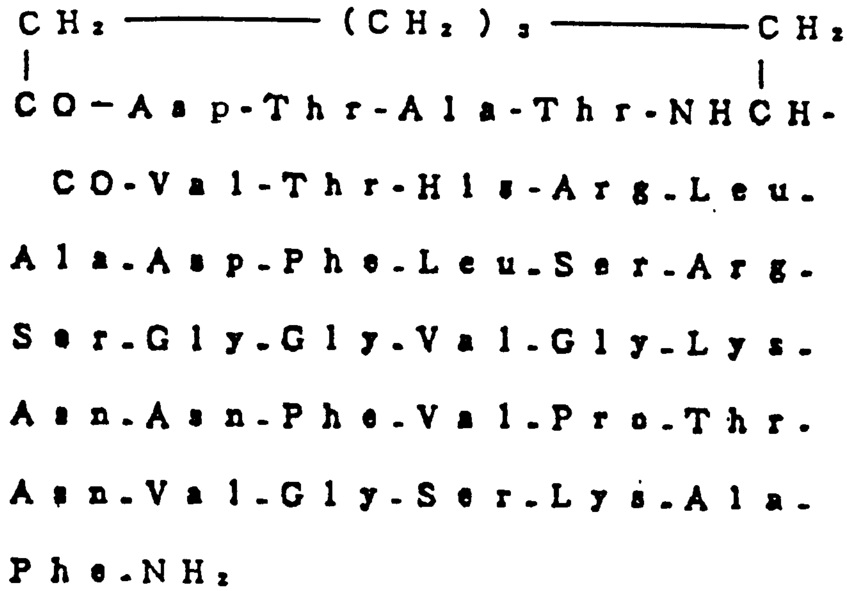

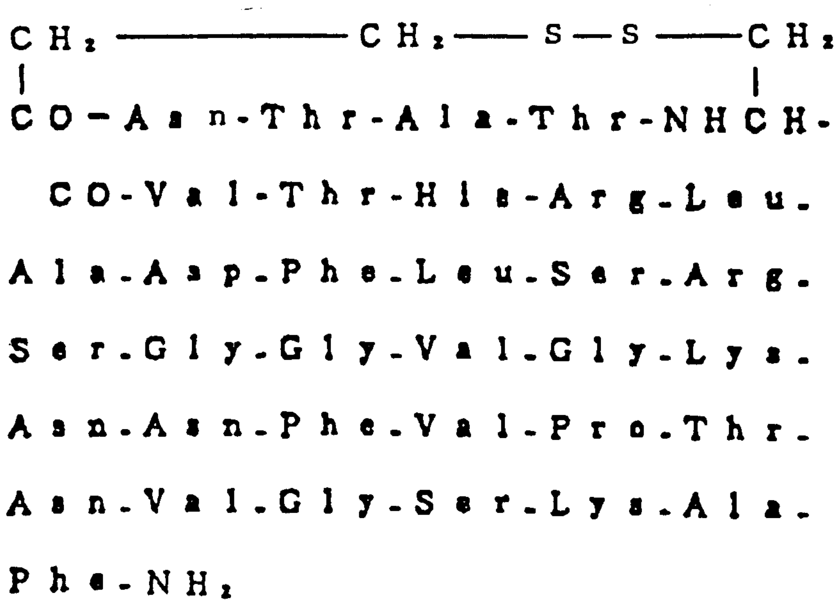

The present invention also provides a process for the preparation of a compound of formula (1) wherein Y is sulfur and A is Asn or Asp, or a physiologically acceptable salt thereof, which process comprises:

(i) synthesising a peptide which has a chain structure of the formula:

$Q_1$-S-$(CH_2)_2$-CO-A-Thr-Ala-Thr-Cys-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-Val-Gly-Ser-Lys-Ala-Phe-$Q_2$

wherein A is Asn or Asp, $Q_1$ is hydrogen or a mercapto-protecting group and $Q_2$ is optional but, if present, is - $NH_2$ or together with the carboxyl group from -Phe- represents a protected amide group, and in which side-chain functional groups are optionally protected;

(ii) when Q is a mercapto-protecting group and/or when the side-chain mercapto group of Cys is protected, removing the or each said protecting group;

(iii) oxidising the free mercapto groups to form a disulfide bridge;

(iv) removing any side-chain functional group-protecting groups optionally present and, when $Q_2$ is not

$-NH_2$, converting the terminal carboxy group of Phe into an amide group; and

(v) if desired, converting the resulting compound of formula (1) into a physiologically acceptable salt thereof.

The peptide employed in step (i) may be prepared by, for example, solid phase synthesis or solution phase synthesis.

The present invention also provides a process for the preparation of a compound of formula (1) wherein Y is methylene or a physiologically acceptable salt thereof, which process comprises:

(i) synthesising either a peptide which has a chain structure of the formula:

**A-Thr-Ala-Thr-Asu-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-Val-Gly-Ser-Lys-Ala-Phe-$Q_2$**

wherein A and $Q_2$ are as defined above, and in which side-chain groups are optionally protected, or a part thereof comprising the sequence:

A-Thr-Ala-Thr-Asu-;

(ii) cyclising the sequence A-Thr-Ala-Thr-Asu to form thereby the unit

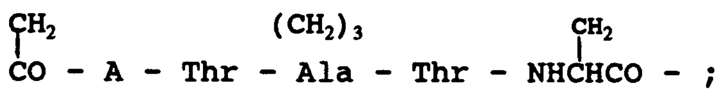

(iii) synthesising the remainder of the sequence of the peptide of formula (1) when not synthesised in step (i);

(iv) removing any side-chain functional group-protecting groups optionally present and, when $Q_2$ is not $-NH_2$, converting the terminal carboxy group of -Phe- into an amide group; and

(v) if desired, converting the resulting compound of formula (1) into a physiologically acceptable salt thereof.

The present invention further provides a process for the preparation of a compound of formula (1) wherein Y is methylene or a physiologically acceptable salt thereof, which process comprises:

(i) synthesising a part of the compound of formula (1) which has the structure:

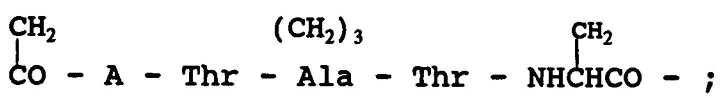

wherein A is as defined above, and in which side-chain functional groups are optionally protected;

(ii) synthesising the other part of the compound of formula (1), optionally providing protecting groups for side-chain functional groups;

(iii) condensing the parts synthesised in steps (i) and (ii);

(iv) removing any side-chain functional group-protecting groups optionally present and, if the carboxy-terminal -Phe- carboxy group has not been converted into an amide group, effecting the said conversion; and

(v) if desired, converting the resulting compound of formula (1) into a physiologically acceptable salt thereof.

A peptide (1) of the present invention can be synthesized by a known conventional process for peptide synthesis.

(1) A process by liquid phase synthesis:

A peptide [1] wherein Y is sulfur is synthesized by converting a C-terminal carboxyl in phenylalanyl to an amide, condensating successively a protected amino acid and/or a protected lower peptide in the order

of the amino acid sequence shown in formula [1], removing protecting groups for L-cystinyl and mercapto in β-mercaptopropionic acid and protecting groups for functional groups in the other side chain by acid hydrolysis, and oxidizing mercapto to form a disulfide bridge at an final stage of the condensation reaction.

A peptide [1] wherein Y is methylene is synthesized by converting a C-terminal carboxyl in phenylalanyl to an amide, condensating successively a protected amino acid and/or a protected lower peptide in the order of the amino acid sequence shown in formula [1], subjecting the constructing unit containing the fragment of formula

-A-Thr-Ala-Thr-
    $(CH_2)_5COOR$
    |
-NHCHCO-

wherein R is an activated ester residue and A is as defined above to a cyclization reaction at a required stage of the above condensation reaction, and removing the protecting groups of active groups by acid hydrolysis at the final stage of the condensation reaction.

The condensation reaction can be proceeded by repeating the removal and addition of a protecting group and can be one used in a conventional peptide synthesis. The protecting group for a starting material and intermediates used in the process is a known protecting group in peptide chemistry, for example a peptide which can easily removed by a known procedure such as hydrolysis, acid decomposition, reduction, aminolysis or hydrazinolysis. These protecting groups are found in references or commentaries on peptide chemistry.

Examples of preferred protecting groups are butyloxy carbonyl, benzyloxy carbonyl or p-methoxybenzyloxy carbonyl for an α-amino group, benzyloxy carbonyl or p-chlorobenzyloxy carbonyl for a side chain amino group such as ε-amino in lysine, methyl ester or benzylester for an α-carboxyl group, benzyl ester for a side chain carboxyl group such as a side chain carboxyl in aspartic acid, t-butyl ester for a side chain carboxyl in α-aminosuberic acid, benzyl for a hydroxyl group in serine and threonine, and methylene-2-sulfonyl or tosyl for an amino group of guazinino in arginine.

In the synthesis of a peptide [1] of the present invention, condensation of each amino acid and/or lower peptide can be proceeded by reacting an amino acid or a lower peptide having a protected α-amino and activated terminal α-carboxyl group with an amino acid or a lower peptide having a free α-amino and protected terminal carboxyl group or alternatively by reacting an amino acid or a lower peptide having an activated α-amino and protected terminal carboxyl with an amino acid or a lower peptide having a protected terminal carboxyl group.

The carboxyl group can be activated by converting it to, for example, an acid azide, acid anhydride, acid imidazolide or activated ester such as the cyanomethyl ester, p-nitrophenyl ester or N-hydroxysuccinimide ester. It can also be activated by using a condensation reagent, for example carbodiimide such as N,N'-dicyclohexyl-carbodiimide (DCCD), N-ethyl-N'-3-dimethyaminopropyl-carbodiimide or N,N'-carbonyldiimidazole.

Examples of the preferred condensation reaction methods are the azide method, activated ester method, mixed anhydride method and carbodiimide method. In the condensation reaction process, it is preferred to avoid or at least minimizing a racemic reaction; a preferred method is an azide method, activated ester method, Wunsch method [Z. Naturforsch., 21b, 426 (1966)] or Geiger method [Chem. Ber., 103, 788 (1970)].

A peptide [1] can be synthesized using any sequence order. However it is preferred to construct an amino acid sequence by connecting amino acids and/or lower peptides in order from the C-terminal.

A further process for obtaining the peptide [1] from the thus synthesized protected peptide chain depends on the structure of the peptide [1] wherein Y is sulfur or Y is methylene.

A peptide [1] wherein Y is sulfur can be obtained by removing the protecting groups from the protected peptide chain, namely β-mercaptopropionyl pentatriaconta-peptideamide having protected ω-amino, side chain caroboxyl, hydroxyl, guanidino and mercapto groups. These protecting groups are preferably removed by a one step removal with acid hydrolysis using, for example, trifluoromethane sulfonic acid or anhydrous hydrogen fluoride to obtain β-mercaptopropionyl pentatriaconta-peptideamide having a free mercapto group.

In the said peptide-amide an inner molecule disulfide linkage is formed by oxidation to obtain the

peptide [1]. A disulfide linkage can generally be made by oxidation with oxygen in water, diiodo-ethane in an organic solvent, iodine in glacious acetic acid or potassium ferricyanide in water.

In the case that a peptide [1] wherein Y is methylene is obtained, the construction unit of formula

$$-A-Thr-Ala-Thr-,$$

$$\begin{array}{l} \quad (CH_2)_5COOR \\ \qquad | \\ -NHCHCO- \end{array}$$

wherein R and A are as defined above is subjected to a cyclization reaction at any stage of the condensation reaction at the stage of protected peptide chain synthesis. The cyclization can be made by a condensation reaction with an $\omega$-carboxyl group in $\alpha$-aminosuberic acid and a free amino group in an N-terminal amino acid. In the reaction hydroxyl in threonine and the side chain carboxyl in aspartic acid are preferably protected.

The thus obtained cyclic peptide having a protected or unprotected activated group and a large peptide having a protected or unprotected activated group are condensed and if a protected group exists, the protecting group is removed.

Thus pentatriaconta peptide-amide having protected $\epsilon$-amino, side-chain carboxyl, guanidino and hydroxyl groups can be obtained. These protective groups are preferably removed by acid hydrolysis, for example using trifluoromethane sulfonate and anhydrous hydrogen fluoride, in a one step removal method to obtain the peptide [1].

(2) A process by solid phase synthesis:

A solid phase peptide synthesis method can be applied in part or whole to the peptide [1] synthesis.

For example, in case of synthesis of a peptide [1] wherein Y is sulfur, a peptide fragment (3 - 37) [hereinafter a peptide having an amino acid sequence constituting amino acids No. 3 to No. 37 is referred to as peptide fragment (3 - 37) or peptide (3 - 37)] is synthesized by a solid phase method, and an $\alpha$-amino group in the said peptide is acylated with $\beta$-mercaptopropionic acid to obtain a protected pentatriaconta-peptide bound resin. These protecting groups and resin are removed by a known method, for example using trifluoromethane sulfonic acid or anhydrous hydrogen fluoride, in a one step removal to obtain $\beta$-mercaptopropionyl pentatriaconta peptide-amide having a free mercapto group. A peptide [1] can be obtained by constructing an inner molecule disulfide bond as illustrated in the liquid phase synthesis (1) above.

In the case of the synthesis of peptide [1] wherein Y is methylene, a peptide fragment (9 - 37) is synthesized by a solid phase method, and a cyclic peptide fragment containing N-terminal $\alpha$-aminosuberic acid is synthesized by a liquid phase method. Subsequently the above two peptide fragments are condensed in a solid phase method to obtain a protected pentatriaconta peptide bound resin. These protecting groups and resin are removed by a known method, for example using trifluoromethane sulfonic acid or anhydrous hydrogen fluoride, in a one step removal to obtain the peptide [1].

Examples of resins used in the solid phase method are conventional resins such as benzhydrylamine resin or p-methyl-benzhydrylamine resin. A resin of desired functional equivalence or cross-linkage can be obtained or is commercially available.

In the solid phase method amino acids are condensated, one by one, in the order of the amino acid sequence of formula [1], from the C-terminal amino acid to the third amino acid (amino acid No. 3) (in case of a peptide wherein Y is sulfur), or from the C-terminal amino acid to the 9th amino acid (amino acid No. 9) (in case of a peptide wherein Y is methylene). Functional groups in the amino acids are protected by known methods. Examples of protecting groups are as illustrated above.

In the solid phase reaction, a resin in a reaction vessel is swollen by adding dichloromethane, chloroform, dimethylformamide, benzene or a solvent for swelling the resin, in an amount of 2 - 20 ml solvent per 1g resin. In another reaction vessel, 1 - 6 equivalents of t-butyloxycarbonyl- (hereinafter designated as Boc-) amino acid for 1 equivalent of amino group in the resin are previously reacted with DCC, and the obtained acid anhydride, which is separated from the by-product dicyclohexylurea (hereinafter designated as DCU), is added to the above resin. The amount of condensation agent (DCC) is 0.5 - 3 equivalents for 1 equivalent of Boc-amino acid. The reaction generally proceeds for 5 - 60 minutes.

The coupling amount of amino acid or peptide can be determined according to a conventional method [T. Fairwell, et al., Biochemistry, 22: 2691 (1983)] by checking the amount of Boc-amino acid after sampling the Boc-amino acid-resin or Boc-peptide-resin obtained in each process.

The protecting group for the α-amino group, Boc, is removed by an acid such as trifluoroacetic acid and the condensation reaction is performed. An automatic solid phase synthesiser is generally used; however a manual procedure can be applied. All the operation is preferably conducted under a nitrogen atmosphere.

A peptide fragment (3 - 37) or (9 - 37) bound to a resin can be obtained.

A peptide fragment (3 - 37) bound resin is acylated with β-mercaptopropionic acid in the final step to obtain a β-mercaptopropionyl-protected pentatriaconta peptide-amide bound resin.

The thus obtained protected pentatriaconta peptide-amide bound resin is, as previously illustrated, treated with anhydrous hydrogen fluoride to remove the protecting group and resin in one step; β-mercaptopropionyl pentatriaconta peptide-amide having a free mercapto group can be obtained.

A peptide [1] wherein Y is sulfur can be obtained by forming an inner molecule disulfide bond in the above β-mercaptopropionyl pentatriaconta peptide-amide having a free mercapto group.

In the synthesis of a peptide [1] wherein Y is methylene, a peptide fragment (9 - 37) bound resin is condensed with a cyclic peptide fragment containing α-aminosuberic acid.

The thus obtained protected pentatriaconta bound resin containing α-aminosuberic acid is treated with anhydrous hydrogen fluoride to remove the protecting group and resin in one step; the peptide [1] wherein Y is methylene is obtained.

(3) Isolation and purification:

The peptide [1] can be purified by a known conventional purification method for peptide or protein chemistry. For example a gel-filtration method, for example using Sephadex G-25, Sephadex G-50 or Sephadex LH-20 (trade names), column chromatography using ion-exchange resin or carboxy methyl cellulose, or HPLC can be applied.

A peptide [1] of the present invention can be obtained according to the process in the form of a free base or salt. For example a salt with a known organic acid such as a acetic acid can be prepared.

Abbreviations used in the specification and drawings are as follows:

| | |
|---|---|
| Asu: | L-α-aminosuberic acid |
| Asn: | L-asparagine |
| Asp: | L-aspartic acid |
| Ala: | L-alanine |
| Thr: | L-threonine |
| Val: | L-valine |
| His: | L-histidine |
| Arg: | L-arginine |
| Leu: | L-leucine |
| Phe: | L-phenylalanine |
| Ser: | L-serine |
| Gly: | glycine |
| Lys: | L-lysine |
| Pro: | L-proline |
| Boc: | t-butyloxycarbonyl |
| Z: | benzyloxycarbonyl |
| Cl-Z: | p-chlorobenzyloxycarbonyl |
| Bzl: | benzyl |
| OSu: | N-hydroxysuccineimide ester |
| ONp: | p-nitrophenyl ester |
| OMe: | methyl ester |
| OBut: | t-butyl ester |
| OBzl: | benzyl ester |
| TFA: | trifluoroacetic acid |
| Ether: | diethyl ether |
| DMF: | N,N'-dimethylformamide |
| MeOH: | methanol |
| DCM: | dichloromethane |
| DIEA: | diisopropyl ethylamine |

HOBt: 1-hydroxybenzo triazole
MBHA-resin: p-methylbenzhydrylamine resin

The serum calcium and serum phosphate reducing activities of the compounds of the present invention can be shown as follows:

[Assay method]

Desalanyl-deamino-c-CGRP, desalanyl-[$Asu^{2,7}$]-c-CGRP and desalanyl-[$Asp^{3}$,$Asu^{2,7}$]-c-CGRP (each 80 μg), c-CGRP (JP-A-61-273581) and known h-CGRP (each 80 μg for control group) dissolved in citrate buffer, pH 6.5, containing 0.1% bovine serum albumin (hereinafter designated as dissolving medium) (1 ml) were administered intravenously into the tail veins of Wistar rats, body weight 80 - 90 g, 5 - 6 rats in one group, at 20 or 80 μg/kg. After 30 and 60 minutes from administration, blood samples were collected from abdominal descending aorta. The serum calcium concentration was measured by atomic adsorption spectrophotometry. The serum phosphate was measured by the method according to Goldenberg et al. [Clin. Chem., 12: 872 - 882 (1966)].

[Results]

As shown in Fig. 1 and Fig. 2, concentrations of serum calcium and phosphate were reduced by more than 30% in the desalanyl-deamino-c-CGRP, 20 μg/kg, administered group (-O-) as compared with the control group (dissoving medium administered group) (-X-) and these activities were observed to continue after 2 hours. These activities were stronger than that of h-CGRP administered at quadruple amounts (-□-, 80 μg/kg), and were as significantly strong as c-CGRP administered at an equivalent dose (-△-, 20 μg/kg), and the effects were prolonged significantly.

Furthermore as shown in Fig. 1 and Fig. 2, the concentrations of serum calcium and phosphate were reduced by more than 30% in the desalanyl-deamino -c-CGRP, 80 μg/kg, administered group (-●-) as compared with the control group (dissoving medium administered group) (-X-) and these activities were observed to continue after 2 hours. Moreover the serum calcium reducing activity was further enhanced. These activities were stronger than that of h-CGRP (-□-, 80 μg/kg) and c-CGRP (-▲-, 80 μg/kg) administered at an equivalent dose, and the effects were prolonged significantly.

As shown in Fig. 3 and Fig. 4, the concentrations of of serum calcium and phosphate were reduced by more than 30% in the desalanyl-[$Asu^{2,7}$]-c-CGRP, 80 μg/kg (-●-) and the desalanyl-[$Asp^{3}$,$Asu^{2,7}$]-c-CGRP, 80 μg/kg (-■-), administered groups as compared with the control group (dissolving medium administered group) (-X-) and these activities were observed to continue after 2 hours. These activities were stronger than that of h-CGRP administered at an equivalent amount (-□-, 80 μg/kg), and were as significantly strong as c-CGRP administered at an equivalent dose (-△-, 20 μg/kg), and the effects were prolonged significantly.

As illustrated above, the peptide [1] of the present invention has a stronger serum calcium reducing activity and serum phosphate reducing activity as compared with known h-CGRP. Furthermore it has superior sustained activity and has stronger biological activity with prolonged sustained activity as compared with c-CGRP. Moreover it is stable against the action of amino-peptidase in vivo, and hence is useful for the treatment of a calcium metabolic disorder, cardiac disease and ulcers, or for improvement in brain circulatory systems.

The following Examples further illustrate the present invention.

In the examples PF( ) means the peptide fragment having the amino acid sequence of the numbers in the brackets.

The carrier and developers used in thin layer chromatography (TLC) and conditions of hydrolysis in the amino acid analysis are, if not otherwise specified, as follows.

<TLC>

Carrier: silica-gel (Merck, Art 5715)
Developer: 1; chloroform : methanol : acetic acid (95 : 5 : 3)
2; chloroform : methanol acetic acid (85 : 15 : 5)

<Hydrolysis>

Samples were hydrolysed at 110 °C for 24 - 48 hours with HCl in a sealed tube.

Example 1

Production of desalanyl-[Asu$^{2,7}$]-c-CGRP:

Anisole (1 ml) was added to desalanyl-[Asu$^{2,7}$]-protected-c-CGRP (3-37)-MBHA-resin of formula:

```
CH2 ────────── (CH2)3 ────────── CH2
|                                 |
CO-Asn-Thr-Ala-Thr-NHCH-
CO-Val-Thr(Bzl)-His-Ar
g(Tos)-Leu-Ala-Asp(OBz
l)-Phe-Leu-Ser(Bzl)-Ar
g(Tos)-Ser(Bzl)-Gly-Gl
y-Val-Gly-Lys(Cl-Z)-As
n-Asn-Phe-Val-Pro-Thr(
Bzl)-Asn-Val-Gly-Ser(B
zl)-Lys(Cl-Z)-Ala-Phe-
```

-MBHA-resin (1.09 g). Anhydrous hydrogen fluoride (25 ml) was added therein and stirred at 0°C for 1 hour. After distilling off the anhydrous hydrogen fluoride in vacuo, the residue was washed with ether and 0.1 M acetic acid (20 ml) was added to extract the peptide. The extract was passed through a Dowex WGR column (2.8 x 15 cm) (trade name) and eluted with 0.1 M acetic acid (60 ml). The eluate was freeze dried to obtain a white powder (370 mg).

The powder was charged on a column of carboxymethyl cellulose (2.8 x 13 cm) and was subjected to linear gradient elution with 0.01 M aqueous ammonium acetate (pH 4.5, 300 ml)~0.5 M aqueous ammonium acetate (pH 5.3, 300 ml). Samples (100 μl) from the fractions (each 10 ml) were measured by colorimetry by the Folin-Lolly method at 750 nm. Fractions No. 33 - 37 were collected and charged on a column (2.8 x 5.5 cm) of CHP-20 resin (trade name, Mitsubishi Kasei Kogyo), and eluted with linear gradient elution in 0.1 M aqueous acetic acid containing 25% acetonitrile (150 ml)~0.1 M aqueous acetic acid containing 40% acetonitrile (150 ml). Fractions (each 6.4 ml) No. 10 - 12 were collected and freeze dried to obtain a white powder (30.5 mg).

The powder was purifed by reverse phase HPLC to obtain purifed desalanyl-[Asu$^{2,7}$]-c-CGRP (3.4 mg).

| | |
|---|---|
| Column: | Nucleosil $5C_{18}$ |
| Buffer: | 0.1% TFA-acetonitrile (a gradient elution with acetonitrile concentration from 28 to 38% in 25 minutes) |
| Flow: | 2.5 ml/min |
| Fraction: | peak collected at retention time 17.8 min |
| Physical properties: | pI: more than 10.25 |

$$[\alpha]_D^{26.5}:$$

-53.3 (c = 0.094, 0.1 M acetic acid)

Amino acid analysis:

Asp4.96 (5), Thr3.77 (4), Ser2.83 (3), Pro1.12 (1), Gly4.15 (4), Ala3.00 (3), Val3.89 (4), Leu2.00 (2), Phe3.10 (3), Lys2.11 (2), His0.98 (1), Arg2.10 (2), Asu1.12 (1)

Desalanyl-[$Asu^{2,7}$]-protected-c-CGRP-MBHA-resin was obtained by the following procedure.

Solid phase peptide synthesizer: 430-A peptide synthesizer Applied Biosystems Corp.

(1) Production of PF(9-37)-MBHA-resin, i.e.;

H-Thr(Bzl)-His-Arg(Tos)-Leu-Ala-Asp(OBzl)-Phe-Leu-Ser(Bzl)-Arg(Tos)-Ser(Bzl)-Gly-Gly-Val-Gly-Lys(Cl-Z)-Asn-Asn-Phe-Val-Pro-Thr(Bzl)-Asn-Val-Gly-Ser(Bzl)-Lys(Cl-Z)-Ala-Phe-MBHA-resin

MBHA-resin (Applied Biosystems Corp., amino group: 0.61 mM/g) (0.8 g) set in a reaction vessel of a solid phase peptide synthesizer was treated with DCM (8 ml) (4 times, each 1 min), DCM solution (8 ml) containing 60% TFA (20 min), DCM (4 ml) (3 times, each 15 s), DMF solution (3 ml) containing DIEA (1 ml) (2 times, each 1 min) and DMF (8 ml) (6 times, each 40 s), in this order under a nitrogen atmosphere with stirring, and filtered after each treatment.

DCC (0.5 M-DCM solution) (2 ml) was added to Boc-Phe (2 mM, amino acid sequence No. 37) dissolved in DCM (5 ml) in an amino acid activator vessel and reacted for 5 minutes. The filtered reaction mixture was transferred to a concentration vessel and DMF (3 ml) was added therein. Distilled DCM was then distilled off under a nitrogen atmosphere. Further DMF (3 ml) was added and the mixture was transferred to the above reaction vessel, then subjected to reaction for 25 minutes. The reaction mixture was washed 6 times with DCM (8 ml) for each 20 seconds, and filtered to obtain a Boc-Phe-MBHA-resin.

The Boc-Phe-MBHA-resin was washed 4 times with DCM (8 ml, each 1 min) in the reaction vessel and

filtered. 40% DCM solution (8 ml) containing 60% TFA was added thereto and stirred for 20 minutes to remove the Boc. The thus obtained resin was washed 3 times with DCM (4 ml, each 15 s), 2 times with DMF solution (3 ml) containing DIEA (1 ml) (each 1 min) and 6 times with DMF (8 ml, each 40 s), in this order, and filtered.

DCC (0.5 M-DCM solution) (2 ml) was added to Boc-Ala (2 mM, amino acid sequence No. 36) dissolved in DCM (5 ml) in an amino acid activator vessel, reacted for 5 minutes, then treated as same as the Boc-Phe above. The reaction mixture was concentrated after adding DMF under a nitrogen atmosphere, transferred to a reaction vessel and reacted for 20 minutes, washed 6 times with DCM (8 ml, each 20 s) and filtered to obtain a Boc-Ala-Phe-MBHA-resin.

Subsequently, amino acids (sequence from No. 35 to 9) were subjected to coupling reactions to obtain a PF(9-37)-MBHA-resin.

Protected amino acids used in the process are as follows:

| Amino acid No. | Protected amino acid | amount used (mM) |
|---|---|---|
| 35 | Boc-Lys (Cl-Z) | 2 |
| 34 | Boc-Ser (Bzl) | 2 |
| 33 | Boc-Gly | 2 |
| 32 | Boc-Val | 2 |
| 31 | Boc-Asn | 2x2 |
| 30 | Boc-Thr (Bzl) | 2 |
| 29 | Boc-Pro | 2 |
| 28 | Boc-Val | 2 |
| 27 | Boc-Phe | 2 |
| 26 | Boc-Asn | 2x2 |
| 25 | Boc-Asn | 2x2 |
| 24 | Boc-Lys (Cl-Z) | 2 |
| 23 | Boc-Gly | 2 |
| 22 | Boc-Val | 2 |
| 21 | Boc-Gly | 2 |
| 20 | Boc-Gly | 2 |
| 19 | Boc-Ser (Bzl) | 2 |
| 18 | Boc-Arg (Tos) | 2x2 |
| 17 | Boc-Ser (Bzl) | 2 |
| 16 | Boc-Leu | 2 |
| 15 | Boc-Phe | 2 |
| 14 | Boc-Asp (OBzl) | 2 |
| 13 | Boc-Ala | 2 |
| 12 | Boc-Leu | 2 |
| 11 | Boc-Arg (Tos) | 2x2 |
| 10 | Boc-His (Tos) | 2 |
| 9 | Boc-Thr (Bzl) | 2 |

In the above solid phase synthesis, when Asn and Arg were used, DCC solution (2 ml) and HOBt solution (0.5 M-DMF solution (2 ml) were added to the amino acids (2 mM) in a mixture of DMF-DCM (3 : 1) (4 ml), reacted for 1 minute, then treated the same as the other amino acids. The mixture was transferred to a reaction vessel to perform a coupling reaction, washed with DCM and filtered. Further DCC solution (2 ml) and HOBt solution (0.5 M-DMF solution) (2 ml) were added again to the amino acids (2 mM) in a mixture of DMF-DCM (3 : 1) (4 ml), reacted for 25 minutes, and the mixture was transferred to a reaction vessel to perform a coupling reaction, according to the so-called double coupling method.

(2) Production of desalanyl-[$Asu^{2,7}$]-protected-c-CGRP (3-37)-MBHA-resin:

4 N-hydrogen chloride (0.93 ml) in dioxane was added under cooling at -40 °C to cyclic PF(3-8)[10] of formula:

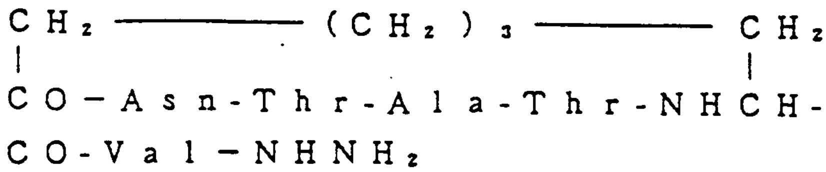

(220 mg) dissolved in DMF (10 ml), and isoamylnitrite (60 µl) at -30 °C thereto. After 30 minutes, confirming the negative hydrazine test, triethylamine (520 µl) was added under cooling at -70 °C to neutralize the reaction mixture. PF(9-37)-MBHA-resin (1.13g) was added therein, triethylamine (90 µl) was further added, stirred at -20 °C~-10 °C for 5 hours, then stirred at 4 °C overnight. The reaction mixture was filtered by suction washed with DMF (10 ml), 0.1 M acetic acid (10 ml) and ethanol (10 ml), in this order, and dried in vacuo to obtain a desalanyl-[$Asu^{2,7}$]-protected-c-CGRP (3-37)-MBHA-resin (1.09 g).

A cyclic PF(3-8) [10] as above was prepared by the following method.

(3) Production of cyclic-protected PF(3-8):

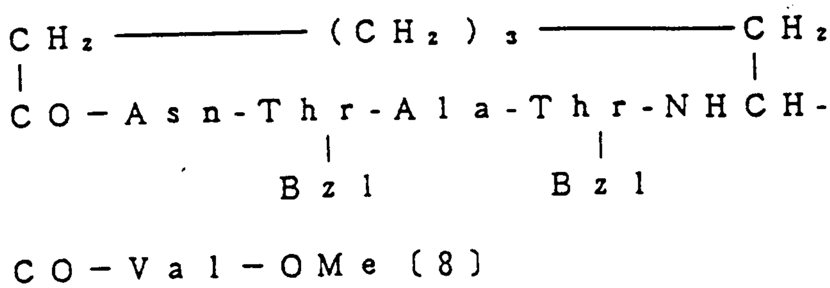

[8]

p-nitrophenyl trifluoroacetate (5.7 equivalents) was added to Boc-Asn-Thr(Bzl)-Ala-Thr(Bzl)-Asu-Val-OMe [7] (3.4 g) dissolved in pyridine (50 ml) and stirred at 45 °C for 4 hours. After distilling off pyridine, ether was added thereto and the precipitated material was collected. Boc was removed by adding TFA (30 ml), which was distilled off in vacuo. Ether was added thereto and the precipitate was collected. The precipitate dissolved in DMF (82 ml) was added dropwise in pyridine (2.3 l) at 45 °C, and stirred at 50 °C for 6 hours and at room temperature overnight. Pyridine was distilled off in vacuo. 0.5% aqueous sodium bicarbonate (100 ml) was added to the residue, and the thus formed precipitate was washed with water completely, then dried in a dessicator to obtain cyclic protected PF(3-8) [8] (3.26 g).

(4) Cyclic PF(3-8):

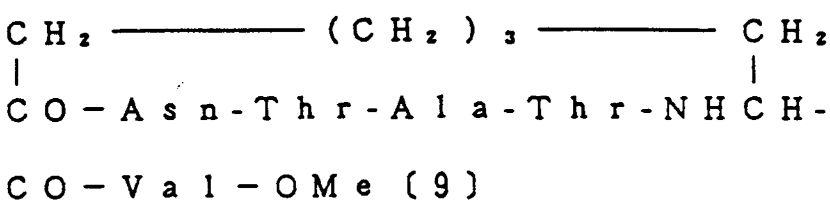

[9]

Anisole (1.5 ml) was added to the cyclic protected PF(3-8) [8] (1.48 g). Anhydrous hydrogen fluoride (15 ml) was added therein and stirred at 0 °C for 1 hour. After the anhydrous hydrogen fluoride was distilled off in vacuo, the residue was washed with ether to obtain a white powder (1.15 g). 0.1 M acetic acid (9 ml) was added to the powder dissolved in acetic acid (3 ml) and charged on a column of CHP-20 (3.2 x 25 cm), packed with 0.1 M acetic acid containing 33% DMF and eluted with linear gradient elution in 33% acetic acid (700 ml)~ 33% acetic acid containing 70% DMF (700 ml).

Fractions (each 10 ml) No. 32 - 49 were collected, the DMF was distilled off and the fractions were freeze dried to obtain a white powder cyclic PF(3-8) [9] (600 mg).

Amino acid analysis:

Asp0.99(1)、Thr1.95(2)、
Ala1.00(1)、Val1.01(1)、
Asu1.07(1)

(5) Cyclic PF(3-8) [10]

Cyclic PF(3-8) [9] (600 mg) in THF (20 ml) was dissolved under heating at 60 ° C and kept at 30 ° C. $NH_2NH_2 \cdot H_2O$ (2 ml) was added thereto and stirred at room temperature for 6 hours. THF (20 ml) and DMF (10 ml) were added therein and stirred overnight. After the solvent was distilled off in vacuo, the residue was dissolved in acetic acid (5 ml), water (15 ml) was added thereto, and charged on a column of CHP-20 (2.8 x 16.0 cm), and eluted with linear gradient elution in 0.1 M acetic acid (300 ml)~0.1M acetic acid containing 40% acetonitrile (300 ml). Fractions (each 9.6 ml) No. 46 - 56 were collected, and freeze dried to obtain a white powder cyclic PF(3-8) [10] (224 mg).

The above Boc-Asn-Thr(Bzl)-Ala-Thr(Bzl)-Asu-Val-OMe [7] was produced by a process illustrated in the production process chart for a peptide fragment (3-8) in Fig. 5.

The physico-chemical properties of the intermediate peptide fragments are illustrated as follows:

(1) PF (5-6) ; Boc-Ala-Thr(Bzl)-OBzl

TLC ; $Rf_1$ ; 0.93

m.p. ; 89-95 °C

Amino acid analysis: Thr 1.00 (1)、Ala 1

$[\alpha]_D^{26.5}$ 10.75 (c=0.99, DMF)

(2) PF (4-6) ; Boc-Thr(Bzl)-Ala-Thr(Bzl)-OBzl

TLC ; $Rf_1$ ; 0.73

m.p. ; 92-94 °C

Amino acid analysis: Thr 1.97 (2)、Ala 1

$[\alpha]_D^{26.5}$ 5.42 (c=1.00, DMF)

(3) PF (4-6) ; Boc-Thr(Bzl)-Ala-Thr(Bzl)-OH

TLC; $Rf_1$ ; 0.32

m.p. ; 54-58 °C

Amino acid analysis:

Thr1.86 (2)、Ala1

$[\alpha]_D^{26.5}$ 24.20 (c=1.03, DMF

(4) PF (7-8) ; Z-Asu (OBut) -Val-OMe

TLC; $Rf_1$ ; 0.90

m.p.: oily at room temperature

(5) PF (7-8) ; H-Asu (OBut) -Val-OMe

TLC; $Rf_1$ ; 0.25

m.p.: oily at room temperature

(6) PF (4-8) ; Boc-Thr (Bzl) -Ala-Thr (Bzl) -Asu (OBut) -Val-OMe

TLC; $Rf_1$ ; 0.64

m.p. ; 107-113 °C

Amino acid analysis:

Thr1.83 (2)、Ala1.00 (1)、Val1.05 (1)、Asu1.18 (1)

$[\alpha]_D^{26.5}$ 5.46 (c=1.03, DMF)

(7) PF (3-8) [7] ;

TLC; $Rf_2$ ; 0.54

m.p. ; 216-218 ℃

Asp1.00 (1), Thr1.92 (2),

Ala1.00 (1), Val1.00 (1),

Asu1.06 (1)

Example 2

Production of desalanyl-[$Asp^3$, $Asu^{2,7}$]-c-CGRP:

Anisole (1 ml) was added to desalanyl-[$Asp^3$, $Asu^{2,7}$]-protected-c-CGRP (3-37)-MBHA-resin of the formula:

$CH_2$ ——— $(CH_2)_3$ ——— $CH_2$
|                                                  |
CO-Asp-Thr-Ala-Thr-NHCH-
CO-Val-Thr(Bzl)-His-Ar
g(Tos)-Leu-Ala-Asp(OBz
l)-Phe-Leu-Ser(Bzl)-Ar
g(Tos)-Ser(Bzl)-Gly-Gl
y-Val-Gly-Lys(Cl-Z)-As
n-Asn-Phe-Val-Pro-Thr(
Bzl)-Asn-Val-Gly-Ser(B
zl)-Lys(Cl-Z)-Ala-Phe-

-MBHA-resin (1.09 g). Anhydrous hydrogen fluoride (25 ml) was added therein and stirred at 0°C for 1 hour. After the anhydrous hydrogen fluoride was distilled off in vacuo, the residue was washed with ether and 0.1 M acetic acid (20 ml) was added therein to extract the peptide. The extract was passed through a Dowex WGR column (2.8 x 16 cm) (trade name) and eluted with 0.1 M acetic acid (60 ml). The eluate was freeze dried to obtain a white powder (410 mg).

The powder was charged on a column of carboxymethyl cellulose (2.8 x 14 cm) and subjected to linear gradient elution with 0.01 M aqueous ammonium acetate (pH 4.5, 300 ml)~0.5 M aqueous ammonium acetate (pH 5.9, 300 ml). Samples (100 μl) from the fractions (each 10 ml) were measured by colorimetry by the Folin-Lolly method at 750 nm. Fractions No. 29 - 31 were collected and charged on a column (2.8 x 6.5 cm) of CHP-20 resin (trade name, Mitsubishi Kasei Kogyo), and eluted with linear gradient elution in 0.1 M aqueous acetic acid containing 25% acetonitrile (150 ml)~0.1 M aqueous acetic acid containing 40% acetonitrile (150 ml). Fractions (each 6.4 ml) No. 10 - 12 were collected and freeze dried to obtain a white powder (18.2 mg).

The powder was purifed by reverse phase HPLC to obtain purifed desalanyl-[$Asp^3$, $Asu^{2,7}$]-c-CGRP (2.8 mg).

| | |
|---|---|
| Column: | Nucleosil $5C_{18}$ |
| Buffer: | 0.1% TFA-acetonitrile (a gradient elution with acetonitrile concentration from 28 to 38% in 25 minutes) |

| | |
|---|---|
| Flow: | 2.5 ml/min |
| Fraction: | collected a peak at retention volume 17.7 min |
| Physical properties: | pI: more than 10.25 |

$$[\alpha]_D^{26.5}: -56.8\ (c = 0.091,\ 0.1\ M\ \text{acetic acid})$$

Amino acid analysis:

Asp4.91 (5)、Thr3.80 (4)、
Ser2.80 (3)、Pro1.03 (1)、
Gly4.05 (4)、Ala3.00 (3)、
Val3.80 (4)、Leu1.99 (2)、
Phe3.08 (3)、Lys2.08 (2)、
His0.92 (1)、Arg2.03 (2)、
Asu1.09 (1)

Desalanyl-[Asp$^3$, Asu$^{2,7}$]-protected-c-CGRP-MBHA-resin was obtained by the following procedure.

(1) PF(9-37)-MBHA-resin was produced by the same procedure as in Example 1.

(2) Production of desalanyl-[Asp$^3$, Asu$^{2,7}$]-protected-c-CGRP (3-37)-MBHA-resin:

4 N-hydrogen chloride (0.93 ml) in dioxane was added under cooling at -40 °C to cyclic PF(3-8) [14] of formula:

$CH_2$ ———— $(CH_2)_3$ ———— $CH_2$
|                                                  |
CO−Asp-Thr-Ala-Thr-NHCH-
CO-Val−$NHNH_2$

[14]

(250 mg) dissolved in DMF (10 ml), and isoamylnitrite (60 $\mu$l) at -30 °C thereto. After 30 minutes, confirming the negative hydrazine test, triethylamine (520 $\mu$l) was added under cooling at -70 °C to neutralize the reaction mixture. PF(9-37)-MBHA-resin (1.13g) was added therein, triethylamine (90 $\mu$l) was further added, stirred at -20 °C~-10 °C for 5 hours, then stirred at 4 °C overnight. The reaction mixture was filtered by suction, washed with DMF (10 ml), 0.1 M acetic acid (10 ml) and ethanol (10 ml), in this order, and dried in vacuo to obtain a desalanyl-[Asp$^3$, Asu$^{2,7}$]-protected-c-CGRP (3-37)-MBHA-resin (1.09 g).

The cyclic PF(3-8) [14] above was prepared by the following method:

(3) Production of cyclic protected PF(3-8):

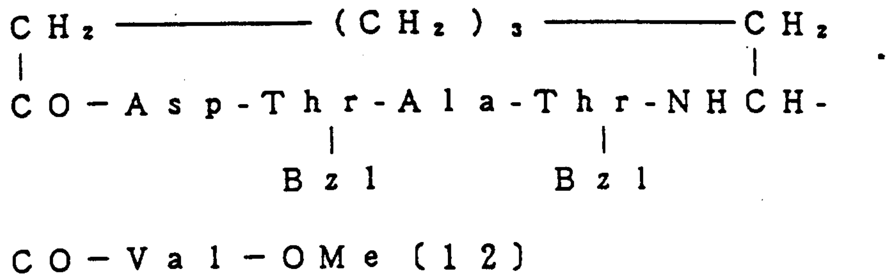

(12)

p-nitrophenyl trifluoroacetate (7 equivalents) was added to Boc-Asp(OBzl)-Thr(Bzl)-Ala-Thr(Bzl)-Asu-Val-OMe [11] (3.2 g) dissolved in pyridine (40 ml) and stirred at 45 °C for 3 hours. After distilling off pyridine, ether was added thereto and the precipitated material was collected. The Boc was removed by adding TFA (30 ml), which was distilled off in vacuo. Ether was added thereto and the precipitate was collected. The precipitate dissolved in DMF (70 ml) was added dropwise in pyridine (2 l) at 45 °C, and stirred at 50 °C for 7 hours and at room temperature overnight. Pyridine was distilled off in vacuo. The residue was extracted with chloroform (400 ml) to obtain cyclic protected PF(3-8) [12] (2.62 g).

(4) Cyclic PF(3-8):

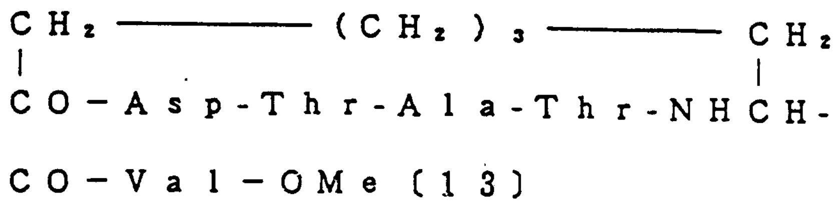

(13)

Anisole (1 ml) was added to the cyclic protected PF(3-8) [12] (2.67 g). Anhydrous hydrogen fluoride (15 ml) was added therein under cooling at 0 °C and stirred for 1 hour. After the anhydrous hydrogen fluoride was distilled off in vacuo, the residue was washed with ether to obtain a white powder (1.75 g). 0.1 M acetic acid (10 ml) was added to the powder dissolved in DMF (10 ml) and charged on a column of CHP-20 (3.2 x 32 cm), packed with 0.1 M acetic acid containing 20% DMF and eluted with linear gradient elution in 0.1 M acetic acid (500 ml) containing 20% DMF~0.1 M acetic acid containing 66% DMF (500 ml). Fractions (each 14.8 ml) No. 54 - 63 were collected, and dried in vacuo to obtain a white powder cyclic PF(3-8) [13] (670 mg).

m.p.: 154 - 160 °C

Amino acid analysis:

Asp0. 98 (1) 、Thr1. 90 (2) 、

Ala1. 00 (1) 、Val0. 96 (1) 、

Asu1. 07 (1)

Mass spectrum: 673($M^+$) (theoretical value 672.71)

(5) Cyclic PF(3-8) [14]

Cyclic PF(3-8) [13] (800 mg) in THF (25 ml) was dissolved under heating and cooled to 30 °C. $NH_2NH_2 \cdot H_2O$ (2 ml) was added thereto and stirred at room temperature for 6 hours. DMF (10 ml) was further added therein and stirred overnight. After the solvent was distilled off vacuo, the residue was dissolved in 0.1 M acetic acid (15 ml), charged on a column of CHP-20 (2.6 x 13.0 cm), and eluted with linear gradient elution in 0.1 M acetic acid (300 ml)~0.1M acetic acid containing 33% acetonitrile (300 ml). Fractions (each 7.9 ml) No. 26 - 40 were collected, and freeze dried to obtain a white powder cyclic PF(3-8) [14] (502 mg).

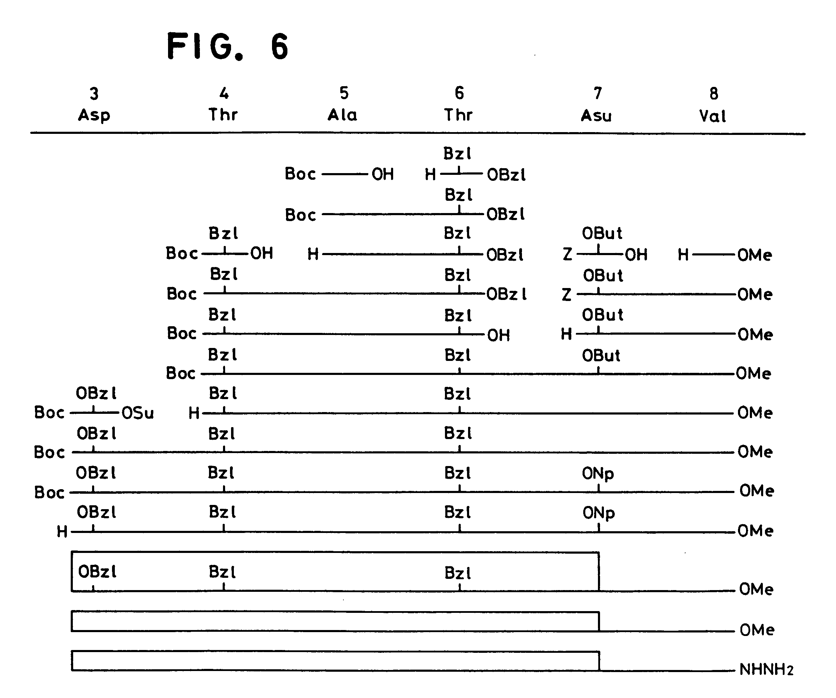

The above Boc-Asp(OBzl)-Thr(Bzl)-Ala-Thr(Bzl)-Asu-Val-OMe [11] was produced by the process illustrated in the production process chart for a peptide fragment (3-8) in Fig. 6.

The physico-chemical properties of the peptide fragment [11] are as follows:

TLC: $Rf_2$; 0.58

Amino acid analysis: Asp 1.04(1), Thr 2.04(2), Ala 1.00(1), Val 1.06 (1), Asu 1.25 (1)

Example 3

Production of desalanyl-deamino-c-CGRP:

Anisole (4 ml), dimethylsulfide (4 ml) and ethanedithiol (0.8 ml) were added to a protected-desalanyl-deamino-c-CGRP-MBHA-resin of formula:

MBzl-S-(CH₂)₂

-CO-Asn-Thr(Bzl)-Ala-Thr(Bzl)-Cys(MBzl)-Val-Thr(Bzl)-His-Arg(Tos)-Leu-Ala-Asp(OBzl)-Phe-Leu-Ser(Bzl)-Arg(Tos)-Ser(Bzl)-Gly-Gly-Val-Gly-Lys(Cl-Z)-Asn-Asn-Phe-Val-Pro-Thr(Bzl)-Asn-Val-Gly-Ser(Bzl)-Lys(Cl-Z)-Ala-Phe-MBHA—resin

(2.65 g). Anhydrous hydrogen fluoride (40 ml) was added therein and stirred at 0 °C for 1 hour. After anhydrous hydrogen fluoride was distilled off in vacuo, the residue was washed with ether and 20% acetic acid (50 ml) was added therein to extract the peptide. The extract was passed through a Dowex WGR column (2.5 x 15 cm) (trade name) and eluted with 1 M acetic acid (160 m). The eluate was freeze dried to obtain a white powder (780 mg). The powder (150 mg) was dissolved in 50 mM $Na_2HPO_4$ buffer (pH 7.5, 10 ml) containing 8 M urea and 5 mM dithiothreitol and stirred at room temperature for 1 hour. The reaction mixture was diluted with 50 mM $Na_2HPO_4$ buffer (pH 7.5, 1125 ml) and an aqueous solution of 20 mM $K_3Fe(CN)_6$ (8 ml) was added thereto.

The solution was charged on a column of CHP-20P resin (trade name, Mitsubishi Kasei Kogyo) (2.5 x 10 cm) and was subjected to linear gradient elution with 0.1 N aqueous formic acid containing 5% acetonitrile (500 ml)~0.1 N aqueous formic acid containing 45% acetonitrile (500 ml).

Samples (100 μl) from the fractions (each 10 ml) were measured by colorimetry by the Folin-Lolly method at 750 nm. Fractions No. 45 - 53 were collected and freeze dried to obtain a white powder product (35 mg). The powder dissolved in 0.1 M acetic acid was charged on a column (1.6 X 45 cm) of Sephadex G-25 Fine, and eluted with 0.1 M aqueous acetic acid. Fractions (each ml) No.5-15 were collected and freeze dried to obtain a white powder product (31 mg).

The powder was purifed by reverse phase HPLC hereinbelow to obtain purifed desalanyl-deamino-c-CGRP (10.0 mg).

| | |
|---|---|
| Column: | YMC-GEL ODS S-5 AM type (20 mmID x 250 mm) |
| Buffer: | 0.1% TFA-acetonitrile (a gradient elution with acetonitrile concentration from 27 to 40% in 30 minutes) |
| Flow: | 7 ml/min |
| Fraction: | a peak collected at retention time 17.3 min |
| Physical properties: | pI: more than 10.25 |
| $[\alpha]_D^{24}$ : | -60.46 (c = 0.086, 0.1 M acetic acid) |

Amino acid analysis (6N-HCl hydrolysate):

| | |
|---|---|
| Asp4.75 (5)、 | Thr3.62 (4)、 |
| Ser2.74 (3)、 | Pro1.01 (1)、 |
| Gly3.85 (4)、 | Ala3.00 (3)、 |
| Val3.70 (4)、 | Leu1.94 (2)、 |
| Phe2.83 (3)、 | Lys2.04 (2)、 |
| His0.95 (1)、 | Arg1.89 (2)、 |

The above protected-desalanyl-deamino-c-CGRP-MBHA-resin was obtained by the following procedure:

A solid phase peptide synthesizer, 430-A peptide synthesizer, Applied Biosystems Corp. was used.

MBHA-resin (Applied Biosystems Corp., amino group: 0.48 mM/g)(1.0 g) set in a reaction vessel of the solid phase peptide synthesizer was treated with DCM (8 ml) (4 times, each 1 min), DCM solution (8 ml) containing 60% TFA (20 min), DCM (4 ml) (3 times, each 15 s), DMF solution (3 ml) containing DIEA (1 ml) (2 times, each 1 min) and DMF (8 ml) (6 times, each 40 s), in this order under a nitrogen atmosphere with stirring and filtered after each treatment.

DCC (0.5 M-DCM solution) (2 ml) was added to Boc-Phe (2 mM, amino acid sequence No. 37) dissolved in DCM (5 ml) in an amino acid activator vessel and reacted for 5 minutes. The filtered reaction mixture was transferred into a concentration vessel, DMF (3 ml) was added therein, then DCM was distilled off under a nitrogen atmosphere. Further DMF (3 ml) was added therein, and the mixture was transferred to the above reaction vessel, then subjected to reaction for 25 minutes. The reaction mixture was washed 6 times with DCM (8 ml) for each 20 seconds, and filtered to obtain a Boc-Phe-MBHA-resin.

The Boc-Phe-MBHA-resin was washed 4 times with DCM (8 ml, each 1 min) in the reaction vessel and filtered 40% DCM solution (8 ml) containing 60% TFA was added thereto and stirred for 20 minutes to remove the Boc. The thus obtained resin was washed 3 times with DCM (4 ml, each 15 s), 2 times with DMF solution (3 ml) containing DIEA (1 ml) (each 1 min) and 6 times with DMF (8 ml, each 40 s), in this order, and filtered.

DCC (0.5 M-DCM solution) (2 ml) was added to Boc-Ala (2 mM, amino acid sequence No. 36) dissolved in DCM (5 ml) in an amino acid activator vessel and reacted for 5 minutes, then treated the same as the Boc-Phe hereinabove. The reaction mixture was concentrated after adding DMF under a nitrogen atmosphere, transferred into a reaction vessel and reacted for 20 minutes, washed 6 times with DCM (8 ml, each 20 s) and filtered to obtain a Boc-Ala-Phe-MBHA-resin.

Subsequently, amino acids (sequence from No. 35 to 3) were subjected to coupling reactions and the peptide was acylated with MBzl-β-mercaptopropionic acid at the final stage of reaction to obtain a protected-desalanyl-deamino-c-CGRP-MBHA-resin.

The protected amino acids used in the process are:

| Amino acid No. | Protected amino acid | amount used (mM) |
|---|---|---|
| 35 | Boc-Lys (Cl-Z) | 2 |
| 34 | Boc-Ser (Bzl) | 2 |
| 33 | Boc-Gly | 2 |
| 32 | Boc-Val | 2 |
| 31 | Boc-Asn | 2x2 |
| 30 | Boc-Thr | 2 |
| 29 | Boc-Pro | 2 |
| 28 | Boc-Val | 2 |
| 27 | Boc-Phe | 2 |
| 26 | Boc-Asn | 2x2 |
| 25 | Boc-Asn | 2x2 |
| 24 | Boc-Lys (Cl-Z) | 2 |
| 23 | Boc-Gly | 2 |
| 22 | Boc-Val | 2 |
| 21 | Boc-Gly | 2 |
| 20 | Boc-Gly | 2 |
| 19 | Boc-Ser (Bzl) | 2 |
| 18 | Boc-Arg (Tos) | 2x2 |
| 17 | Boc-Ser (Bzl) | 2 |
| 16 | Boc-Leu | 2 |
| 15 | Boc-Phe | 2 |
| 14 | Boc-Asp (OBzl) | 2 |
| 13 | Boc-Ala | 2 |
| 12 | Boc-Leu | 2 |
| 11 | Boc-Arg (Tos) | 2x2 |
| 10 | Boc-His (Tos) | 2 |
| 9 | Boc-Thr (Bzl) | 2 |
| 8 | Boc-Val | 2 |
| 7 | Boc-Cys (MBzl) | 2 |
| 6 | Boc-Thr (Bzl) | 2 |
| 5 | Boc-Ala | 2 |
| 4 | Boc-Thr (Bzl) | 2 |
| 3 | Boc-Asn | 2x2 |
| 2 | MBzl-S-$(CH_2)_2$-COOH | 2 |

In the above solid phase synthesis, when Asn and Arg were used, DCC solution (2 ml) and HOBt solution (0.5 M-DMF solution) (2 ml) were added to the amino acids (2 mM) in a mixture of DMF - DCM (3 : 1) (4 ml), reacted for 1 minute, then treated the same as the other amino acids. The reaction mixture was transferred to a reaction vessel to perform a coupling reaction, washed with DCM and filtered. Further DCC solution (2 ml) and HOBt solution (0.5 M-DMF solution) (2 ml) were added again to the amino acids (2 mM) in a mixture of DMF-DCM (3 : 1) (4 ml), reacted for 25 minutes, and the mixture was transferred to a reaction vessel to perform a coupling reaction, according to the so-called double coupling method.

**Claims**

**Claims for the following Contracting States : DE, FR, GB, IT,**

1. A compound of the formula (1):

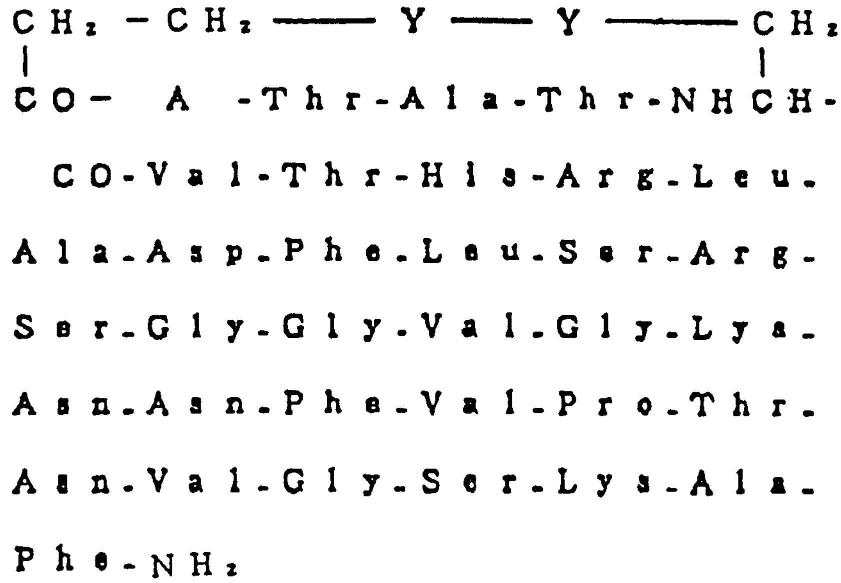

wherein Y is sulphur or methylene and A is Asn or Asp, or a physiologically acceptable salt thereof.

2. A compound according to claim 1 which is a peptide of the formula

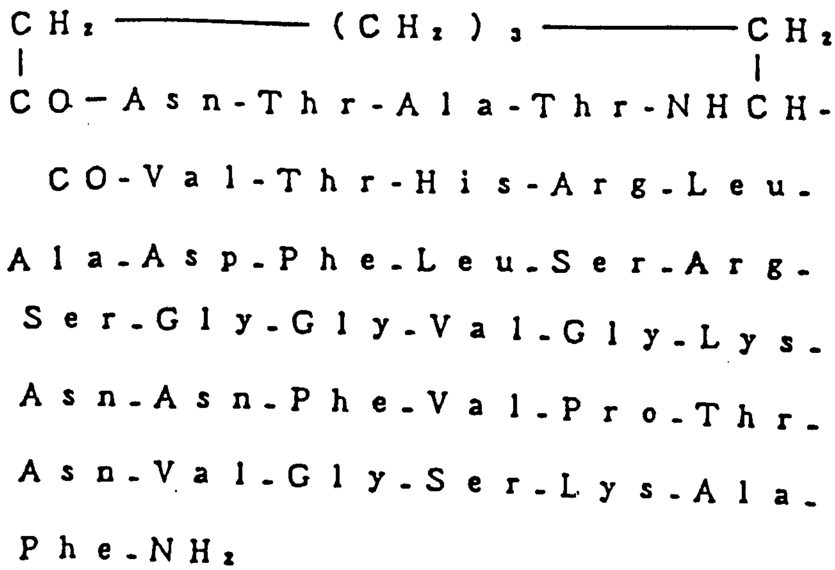

or a physiologically acceptable salt thereof.

3. A compound according to claim 1 which is a peptide of the formula

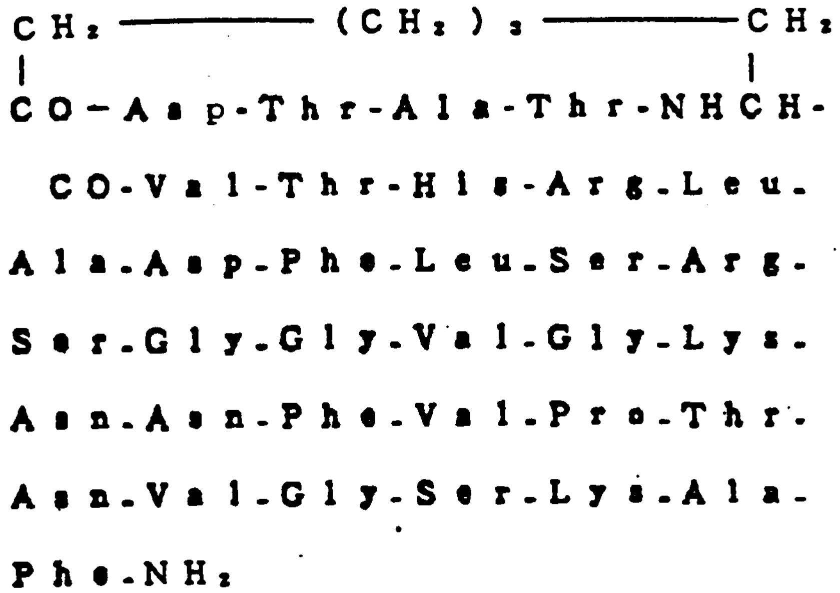

or a physiologicaly acceptable salt thereof.

4. A compound according to claim 1 which is a peptide of the formula

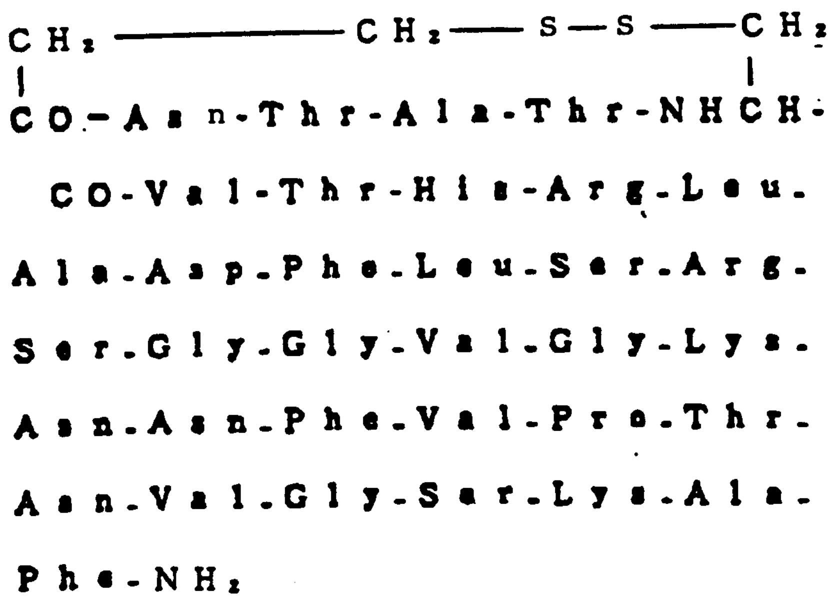

or a physiologically acceptable salt thereof.

5. A process for the preparation of a compound of formula (1) as defined in claim 1 wherein Y is sulfur or a physiologicaly acceptable salt thereof, which process comprises
(i) synthesising a peptide which has a chain structure of the formula

$Q_1$-S-$(CH_2)_2$-CO-A-Thr-Ala-Thr-Cys-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-Val-Gly-Ser-Lys-Ala-Phe-$Q_2$

wherein A is Asn or Asp, $Q_1$ is hydrogen or a mercapto-protecting group and $Q_2$ is optional but, if

present, is $-NH_2$ or together with the carboxyl group from -Phe- represents a protected amide group, and in which side-chain functional groups are optionally protected;
(ii) when Q is a mercapto-protecting group and/or when the side-chain mercapto group of Cys is protected, removing the or each said protecting group;
(iii) oxidising the free mercapto groups to form a disulfide bridge;
(iv) removing any side-chain functional group-protecting groups optionally present and, when $Q_2$ is not $-NH_2$, converting the terminal carboxy group of Phe into an amide group; and
(v) if desired, converting the resulting compound of formula (1) into a physiologically acceptable salt thereof.

**6.** A process according to claim 5, wherein the peptide employed in step (i) has been prepared by solid phase synthesis.

**7.** A process according to claim 5, wherein the peptide employed in step (i) has been prepared by solution phase synthesis.

**8.** A process for the preparation of a compound of formula (1) as defined in claim 1 wherein Y is methylene or a physiologically acceptable salt thereof, which process comprises:
(i) synthesising either a peptide which has a chain structure of the formula:

A-Thr-Ala-Thr-Asu-Val-Thr-His-Arg-Leu-Ala-Asp-Phe-Leu-Ser-
Arg-Ser-Gly-Gly-Val-Gly-Lys-Asn-Asn-Phe-Val-Pro-Thr-Asn-Val-
Gly-Ser-Lys-Ala-Phe-$Q_2$

wherein A and $Q_2$ are as defined in claim 5, and in which side-chain groups are optionally protected, or a part thereof comprising the sequence:

A-Thr-Ala-Thr-Asu-;

(ii) cyclising the sequence A-Thr-Ala-Thr-Asu to form thereby the unit

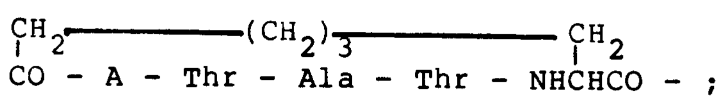

(iii) synthesising the remainder of the sequence of the peptide of formula (1) when not synthesised in step (i);
(iv) removing any side-chain functional group-protecting groups optionally present and, when $Q_2$ is not $-NH_2$, converting the terminal carboxy group of -Phe- into an amide group; and
(v) if desired, converting the resulting compound of formula (1) into a physiologically acceptable salt thereof.

**9.** A process for the preparation of a compound of formula (1) as defined in claim 1 wherein Y is methylene or a physiologically acceptable salt thereof, which process comprises:
(i) synthesising a part of the compound of formula (1) which has the structure

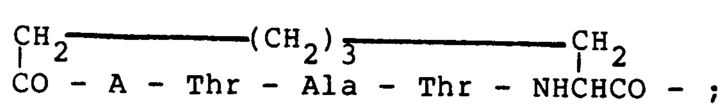

wherein A is as defined in claim 5, and in which side-chain functional groups are optionally protected;
(ii) synthesising the other part of the compound of formula (1), optionally providing protecting groups for side-chain functional groups;

(iii) condensing the parts synthesised in steps (i) and (ii);
(iv) removing any side-chain functional group-protecting groups optionally present and, if the carboxy-terminal -Phe-carboxy group has not been converted into an amide group, effecting the said conversion; and
(v) if desired, converting the resulting compound of formula (1) into a physiologically acceptable salt thereof.

**10.** A pharmaceutical composition comprising a compound of formula (1) as defined in claim 1 or a physiologically acceptable salt thereof as active ingredient and a pharmaceutically acceptable carrier or diluent.

**Claims for the following Contracting State : ES**

**1.** A process for the preparation of a compound of formula (1):

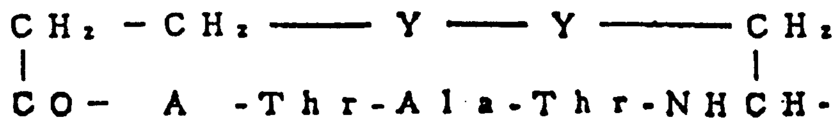

CO-Val-Thr-His-Arg-Leu-
Ala-Asp-Phe-Leu-Ser-Arg-
Ser-Gly-Gly-Val-Gly-Lys-
Asn-Asn-Phe-Val-Pro-Thr-
Asn-Val-Gly-Ser-Lys-Ala-
Phe-$NH_2$

wherein Y is sulfur and A is Asn or Asp, or a physiologically acceptable salt thereof, which process comprises:
(i) synthesising a peptide which has a chain structure of the formula:

$Q_1$-S-$(CH_2)_2$-CO-A-Thr-Ala-Thr-Cys-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-Val-Gly-Ser-Lys-Ala-Phe-$Q_2$

wherein A is Asn or Asp, $Q_1$ is hydrogen or a mercapto-protecting group and $Q_2$ is optional but, if present, is $-NH_2$ or together with the carboxyl group from -Phe- represents a protected amide group, and in which side-chain functional groups are optionally protected;
(ii) when Q is a mercapto-protecting group and/or when the side-chain mercapto group of Cys is protected, removing the or each said protecting group;
(iii) oxidising the free mercapto groups to form a disulfide bridge;
(iv) removing any side-chain functional group-protecting groups optionally present and, when $Q_2$ is not $-NH_2$, converting the terminal carboxy group of Phe into an amide group; and
(v) if desired, converting the resulting compound of formula (1) into a physiologically acceptable salt thereof.

**2.** A process according to claim 1, wherein the peptide employed in step (i) has been prepared by solid

phase synthesis.

3. A process according to claim 1, wherein the peptide employed in step (i) has been prepared by solution phase synthesis.

4. A process for the preparation of a compound of formula (1) as depicted in claim 1 wherein Y is methylene and A is Asn or Asp, or a physiologically acceptable salt thereof, which process comprises:
(i) synthesising either a peptide which has a chain structure of the formula:

**A-Thr-Ala-Thr-Asu-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-Val-Gly-Ser-Lys-Ala-Phe-$Q_2$**

wherein A and $Q_2$ are as defined in claim 1, and in which side-chain groups are optionally protected, or a part thereof comprising the sequence:

A-Thr-Ala-Thr-Asu-;

(ii) cyclising the sequence A-Thr-Ala-Thr-Asu to form thereby the unit

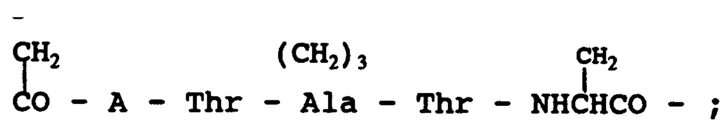

(iii) synthesising the remainder of the sequence of the peptide of formula (1) when not synthesised in step (i);
(iv) removing any side-chain functional group-protecting groups optionally present and, when $Q_2$ is not $-NH_2$, converting the terminal carboxy group of -Phe- into an amide group; and
(v) if desired, converting the resulting compound of formula (1) into a physiologically acceptable salt thereof.

5. A process for the preparation of a compound of formula (1) as depicted in claim 1 wherein Y is methylene and A is Asn or Asp, or a physiologically acceptable salt thereof, which process comprises:
(i) synthesising a part of the compound of formula (1) which has the structure:

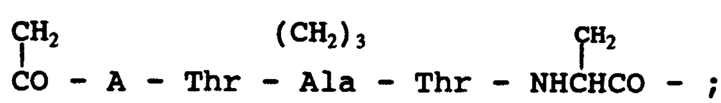

wherein A is as defined in claim 1, and in which side-chain functional groups are optionally protected;
(ii) synthesising the other part of the compound of formula (1), optionally providing protecting groups for side-chain functional groups;
(iii) condensing the parts synthesised in steps (i) and (ii);
(iv) removing any side-chain functional group-protecting groups optionally present and, if the carboxy-terminal -Phe- carboxy group has not been converted into an amide group, effecting the said conversion; and
(v) if desired, converting the resulting compound of formula (1) into a physiologically acceptable salt thereof.

6. A process according to any one of the preceding claims which additionally comprises formulating the obtained compound of formula (1) or physiologically acceptable salt thereof in a pharmaceutical composition with a pharmaceutically acceptable carrier or diluent.

## Patentansprüche

## Patentansprüche für folgende Vertragsstaaten : DE, FR, GB, IT

1. Verbindung der Formel (1):

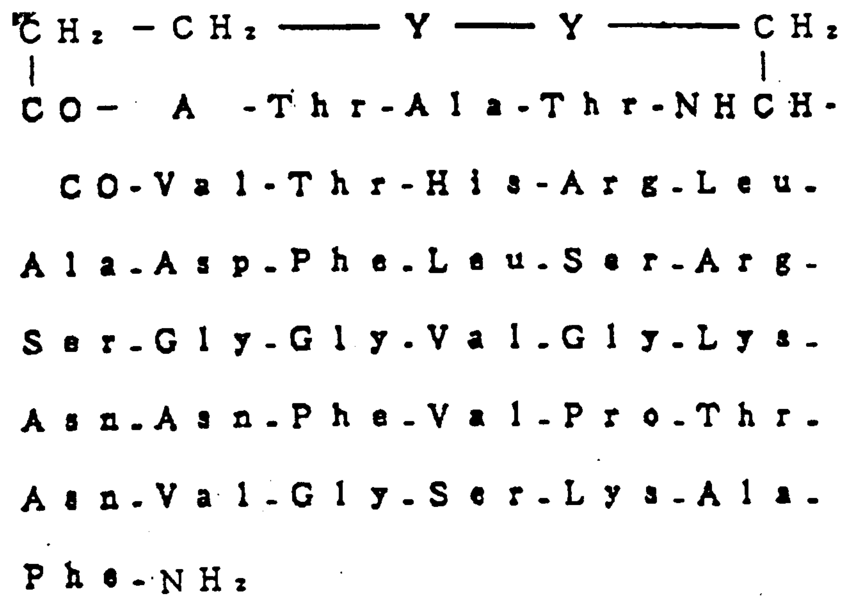

wobei Y die Bedeutung Schwefel oder Methylen und A die Bedeutung Asn oder Asp hat, oder ein physiologisch verträgliches Salz davon.

2. Verbindung nach Anspruch 1 in Gestalt eines Peptids der Formel

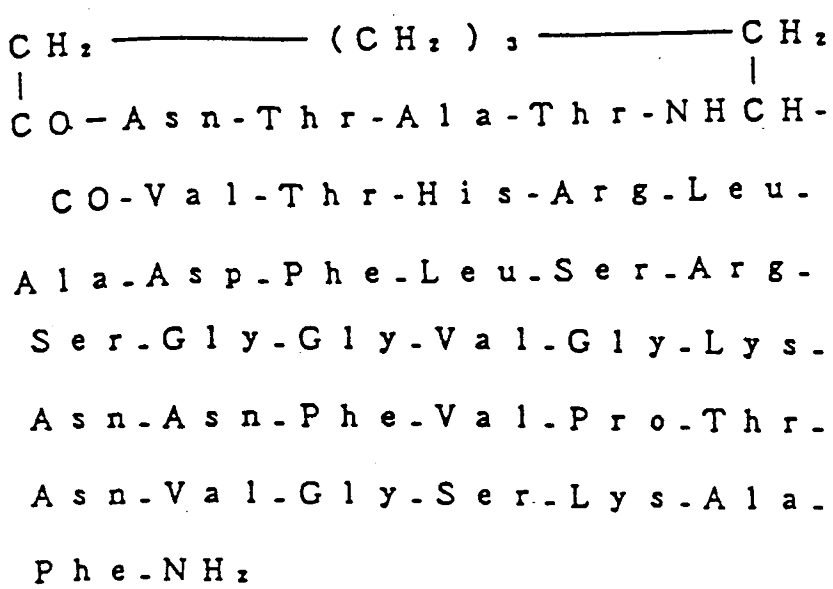

oder eines physiologisch verträglichen Salzes davon.

3. Verbindung nach Anspruch 1 in Gestalt eines Peptids der Formel

$CH_2$ ——— $(CH_2)_3$ ——— $CH_2$
|                                   |
CO–Asp-Thr-Ala-Thr-NHCH-
CO-Val-Thr-His-Arg-Leu-
Ala-Asp-Phe-Leu-Ser-Arg-
Ser-Gly-Gly-Val-Gly-Lys-
Asn-Asn-Phe-Val-Pro-Thr-
Asn-Val-Gly-Ser-Lys-Ala-
Phe-$NH_2$

oder eines physiologisch verträglichen Salzes davon.

**4.** Verbindung nach Anspruch 1 in Gestalt eines Peptids der Formel

$CH_2$ ——— $CH_2$ —— S—S —— $CH_2$
|                                   |
CO–Asn-Thr-Ala-Thr-NHCH-
CO-Val-Thr-His-Arg-Leu-
Ala-Asp-Phe-Leu-Ser-Arg-
Ser-Gly-Gly-Val-Gly-Lys-
Asn-Asn-Phe-Val-Pro-Thr-
Asn-Val-Gly-Ser-Lys-Ala-
Phe-$NH_2$

oder eines physiologisch verträglichen Salzes davon.

**5.** Verfahren zum Herstellen einer Verbindung der Formel (1) gemäß Definition in Anspruch 1, wobei Y die Bedeutung Schwefel hat oder eines physiologisch verträglichen Salzes davon, wobei man
(i) ein Peptid synthetisiert, welches eine Kettenstruktur der Formel

$Q_1$-S-($CH_2$)$_2$-CO-A-Thr-Ala-Thr-Cys-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-Val-Gly-Ser-Lys-Ala-Phe-$Q_2$

aufweist, wobei A die Bedeutung Asn oder Asp hat, $Q_1$ die Bedeutung Wasserstoff oder die einer mercaptoschützenden Gruppe hat und $Q_2$ wahlweise ist, jedoch - falls vorhanden - die Bedeutung -$NH_2$ hat oder zusammen mit der Carboxylgruppe von -Phe- eine geschützte Amidgruppe bedeutet, und wobei funktionelle Seitenkettengruppen wahlweise geschützt sind;
(ii) wenn Q eine mercaptoschützende Gruppe bedeutet und/oder wenn die Seitenketten-Mercaptogruppe von Gys geschützt ist, die oder jede dieser schützenden Gruppen entfernt;
(iii) die freien Mercaptogruppen zur Bildung einer Disulfidbrücke oxidiert;
(iv) alle wahlweise vorhandenen funktionellen gruppenschützenden Gruppen in Seitenketten entfernt und, wenn $Q_2$ nicht -$NH_2$ bedeutet, die terminalen Carboxygruppen von Phe in eine Amidgruppe umwandelt; und
(v) falls gewünscht, die entstehende Verbindung der Formel (1) in ein phyisologisch verträgliches Salz davon umwandelt.

**6.** Verfahren nach Anspruch 5, wobei das in Schritt (i) eingesetzte Peptid mit Hilfe der Festphasensynthese hergestellt worden ist.

**7.** Verfahren nach Anspruch 5, wobei man das in Schritt (i) eingesetzte Peptid mit Hilfe der Lösungsphasensynthese hergestellt worden ist.

**8.** Verfahren zum Herstellen einer Verbindung der Formel (1) gemäß Definition in Anspruch 1, wobei Y die Bedeutung Methylen hat, oder eines physiologisch vertraglichen Salzes davon, wobei man
(i) entweder ein Peptid synthetisiert, welches eine Kettenstruktur der Formel

A-Thr-Ala-Thr-Asu-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-Val-Gly-Ser-Lys-Ala-Phe-$Q_2$

aufweist, wobei A und $Q_2$ die in Anspruch 5 definierte Bedeutung haben, und wobei Seitenkettengruppen wahlweise geschützt sind, oder einen Teil davon, der die Sequenz

A-Thr-Ala-Thr-Asu-

enthält;
(ii) die Sequenz A-Thr-Ala-Thr-Asu zur Bildung der Einheit

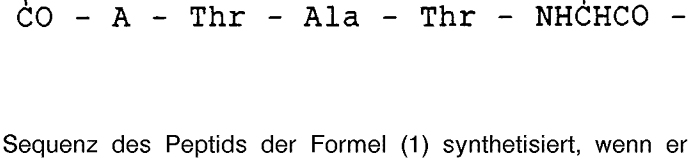

zyklisiert;
(iii) den Rest der Sequenz des Peptids der Formel (1) synthetisiert, wenn er nicht in Schritt (i) synthetisiert wurde;
(iv) alle wahlweise vorhandenen funktionellen gruppenschützenden Gruppen in Seitenketten entfernt und, wenn $Q_2$ nicht -$NH_2$ bedeutet, die terminalen Carboxygruppen von Phe in eine Amidgruppe umwandelt; und
(v) falls gewünscht, die entstehende Verbindung der Formel (1) in ein phyisologisch verträgliches Salz davon umwandelt.

**9.** Verfahren zum Herstellen einer Verbindung der Formel (1) gemäß Definition in Anspruch 1, wobei Y die Bedeutung Methylen hat, oder eines physiologisch verträglichen Salzes davon, wobei man

(i) einen Teil der Verbindung der Formel (1) synthetisiert, der die Struktur

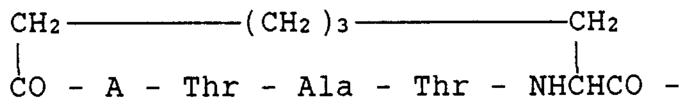

aufweist, wobei A die in Anspruch 5 definierte Bedeutung hat und wobei die funktionellen Seitengruppenketten wahlweise geschützt sind;

(ii) den anderen Teil der Verbindung der Formel (1) synthetisiert und dabei wahlweise Schutzgruppen für funktionelle Seitenkettengruppen vorsieht;

(iii) die in Schritt (i) und (ii) synthetisierten Teile kondensiert;

(iv) alle wahlweise vorhandenen funktionellen gruppenschützenden Gruppen in Seitenketten entfernt und, wenn die carboxyterminale -Phe- Carboxylgruppe nicht in eine Amidgruppe umgewandelt worden ist, die Umwandlung bewirkt; und

(v) falls gewünscht, die entstehende Verbindung der Formel (1) in ein phyisologisch verträgliches Salz davon umwandelt.

**10.** Pharmazeutische Zusammensetzung, enthaltend eine Verbindung der Formel (1) gemäß Definition in Anspruch 1 oder ein physiologisch verträgliches Salz davon als Wirkung und ein(en) pharmazeutisch verträglichen(es) Trägerstoff oder Verdünnungsmittel.

**Patentansprüche für folgenden Vertragsstaat : ES**

**1.** Verfahren zum Herstellen einer Verbindung der Formel(1):

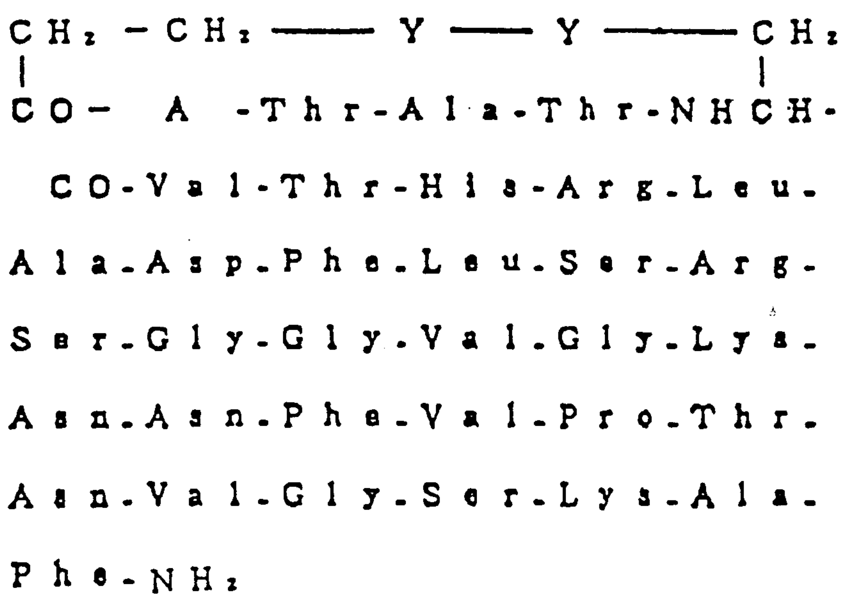

wobei Y die Bedeutung Schwefel hat und A die Bedeutung Asn oder Asp hat oder eines physiologisch verträglichen Salzes davon, wobei man:

(i) ein Peptid synthetisiert, welches eine Kettenstruktur der Formel

$Q_1$-S-$(CH_2)_2$-CO-A-Thr-Ala-Thr-Cys-Val-Thr-His-Arg-
Leu-Ala-Asp-Phe-Leu-Ser-Arg-Ser-Gly-Gly-Val-Gly-
Lys-Asn-Asn-Phe-Val-Pro-Thr-Asn-Val-Gly-Ser-Lys-
Ala-Phe-$Q_2$

aufweist, wobei A die Bedeutung Asn oder Asp hat, $Q_1$ die Bedeutung Wasserstoff oder die einer mercaptoschützenden Gruppe hat und $Q_2$ wahlweise ist, jedoch - falls vorhanden - die Bedeutung $-NH_2$ hat oder zusammen mit der Carboxylgruppe von -Phe- eine geschützte Amidgruppe bedeutet, und wobei funktionelle Seitenkettengruppen wahlweise geschützt sind;
(ii) wenn Q eine mercaptoschützende Gruppe bedeutet und/oder wenn die Seitenketten-Mercaptogruppe von Cys geschützt ist, die oder jede dieser schützenden Gruppen entfernt;
(iii) die freien Mercaptogruppen zur Bildung einer Disulfidbrücke oxidiert;
(iv) alle wahlweise vorhandenen funktionellen gruppenschützenden Gruppen in Seitenketten entfernt und, wenn $Q_2$ nicht $-NH_2$ bedeutet, die terminalen Carboxygruppen von Phe in eine Amidgruppe umwandelt; und
(v) falls gewünscht, die entstehende Verbindung der Formel (1) in ein phyisologisch verträgliches Salz davon umwandelt.

**2.** Verfahren nach Anspruch 1, wobei das in Schritt (i) eingesetzte Peptid mit Hilfe der Festphasensynthese hergestellt worden ist.

**3.** Verfahren nach Anspruch 1, wobei das in Schritt (i) eingesetzte Peptid mit Hilfe der Lösungsphasensynthese hergestellt worden ist.

**4.** Verfahren zum Herstellen einer Verbindung der Formel (1) gemäß Definition in Anspruch 1, wobei Y die Bedeutung Methylen hat und A die Bedeutung Asn oder Asp hat oder eines physiologisch verträglichen Salzes davon, wobei man
(i) entweder ein Peptid synthetisiert, welches eine Kettenstruktur der Formel

A-Thr-Ala-Thr-Asu-Val-Thr-His-Arg-Leu-Ala-Asp-
Phe-Leu-Ser-Arg-Ser-Gly-Gly-Val-Gly-Lys-Asn-Asn-
Phe-Val-Pro-Thr-Asn-Val-Gly-Ser-Lys-Ala-Phe-$Q_2$

aufweist, wobei A und $Q_2$ die in Anspruch 1 definierte Bedeutung haben, und wobei Seitenkettengruppen wahlweise geschützt sind, oder einen Teil davon, der die Sequenz

A-Thr-Ala-Thr-Asu-

enthält;
(ii) die Sequenz A-Thr-Ala-Thr-Asu zur Bildung der Einheit

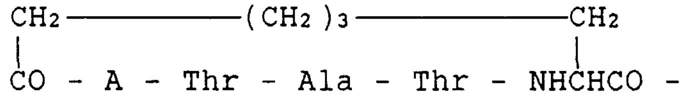

zyklisiert;
(iii) den Rest der Sequenz des Peptids der Formel (1) synthetisiert, wenn er nicht in Schritt (i) synthetisiert wurde;
(iv) alle wahlweise vorhandenen funktionellen gruppenschützenden Gruppen in Seitenketten entfernt und, wenn $Q_2$ nicht $-NH_2$ bedeutet, die terminalen Carboxygruppen von Phe in eine Amidgruppe umwandelt; und

(v) falls gewünscht, die entstehende Verbindung der Formel (1) in ein phyisologisch verträgliches Salz davon umwandelt.

**5.** Verfahren zum Herstellen einer Verbindung der Formel (1) gemäß Definition in Anspruch 1, wobei Y die Bedeutung Methylen hat und A die Bedeutung Asn oder Asp hat oder eines physiologisch verträglichen Salzes davon, wobei man

(i) einen Teil der Verbindung der Formel (1) synthetisiert, der die Struktur

```
CH2 ———————— (CH2)3 ———————— CH2
|                             |
CO - A - Thr - Ala - Thr - NHCHCO -
```

aufweist, wobei A die in Anspruch 1 definierte Bedeutung hat und wobei die funktionellen Seitengruppenketten wahlweise geschützt sind;

(ii) den anderen Teil der Verbindung der Formel (1) synthetisiert und dabei wahlweise Schutzgruppen für funktionelle Seitenkettengruppen vorsieht;

(iii) die in Schritt (i) und (ii) synthetisierten Teile kondensiert;

(iv) alle wahlweise vorhandenen funktionellen gruppenschützenden Gruppen in Seitenketten entfernt und, wenn die carboxyterminale -Phe- Carboxylgruppe nicht in eine Amidgruppe umgewandelt worden ist, die Umwandlung bewirkt; und

(v) falls gewünscht, die entstehende Verbindung der Formel (1) in ein phyisologisch verträgliches Salz davon umwandelt.

**6.** Verfahren nach einem der vorhergehenden Ansprüche, wobei man zusätzlich die erhaltene Verbindung der Formel (1) oder das physiologisch verträgliche Salz davon und einer pharmazeutischen Zusammensetzung mit einem pharmazeutisch verträglichen Trägerstoff oder Verdünnungsmittel formuliert.

## Revendications
## Revendications pour les Etats contractants suivants : DE, FR, GB, IT

**1.** Composé de la formule (1):

```
CH2-CH2————Y————Y————CH2
|                    |
CO- A -Thr-Ala-Thr-NHCH-
CO-Val-Thr-His-Arg-Leu-
Ala-Asp-Phe-Leu-Ser-Arg-
Ser-Gly-Gly-Val-Gly-Lys-
Asn-Asn-Phe-Val-Pro-Thr-
Asn-Val-Gly-Ser-Lys-Ala-
Phe-NH2
```

dans laquelle Y est du soufre ou du méthylène et A est Asn ou Asp, ou un de ses sels physiologiquement acceptables.

**2.** Composé selon la revendication 1, qui est un peptide de la formule

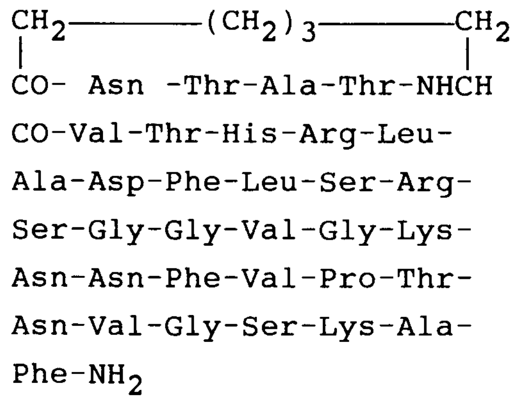

ou un de ses sels physiologiquement acceptables.

**3.** Composé selon la revendication 1, qui est un peptide de la formule

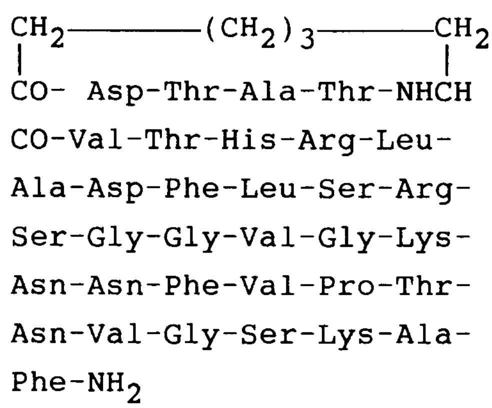

ou un de ses sels physiologiquement acceptables.

**4.** Composé selon la revendication 1, qui est un peptide de la formule

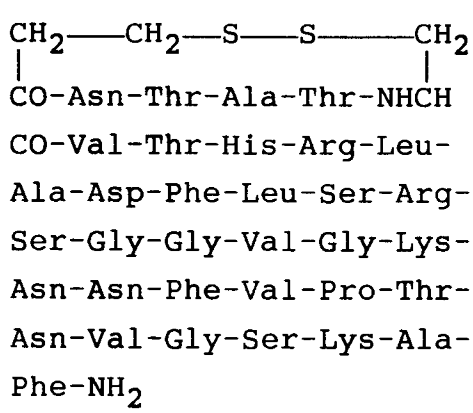

ou un de ses sels physiologiquement acceptables.

**5.** Procédé pour la préparation d'un composé de la formule (1) selon la revendication 1, dans laquelle Y est du soufre, ou d'un de ses sels physiologiquement acceptables, lequel procédé consiste à:
(i) synthétiser un peptide qui a une structure de chaîne de la formule:

$Q_1$-S-$(CH_2)_2$-CO-A-Thr-Ala-Thr-Cys-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-Val-Gly-Ser-Lys-Ala-Phe-$Q_2$,

dans laquelle A est Asn ou Asp, $Q_1$ est de l'hydrogène ou un groupe protecteur de mercapto et $Q_2$ est facultatif, mais, s'il est présent, est $-NH_2$, ou avec le groupe carboxyle en provenance de -Phe- représente un groupe amide protégé, et dans laquelle des groupes fonctionnels de chaîne latérale sont facultativement protégés;

(ii) lorsque Q est un groupe protecteur de mercapto et/ou lorsque le groupe mercapto de chaîne latérale de Cys est protégé, enlever le ou chaque groupe protecteur;

(iii) oxyder les groupes mercapto libres pour former un pont disulfure;

(iv) enlever tous les groupes protecteurs de groupes fonctionnels de chaîne latérale facultativement présents et, lorsque $Q_2$ n'est pas $-NH_2$, convertir le groupe carboxyle terminal de Phe en un groupe amide; et

(v) si on le désire, convertir le composé résultant de la formule (1) en un de ses sels physiologiquement acceptables.

**6.** Procédé selon la revendication 5, dans lequel le peptide employé au stade (i) a été préparé par synthèse en phase solide.

**7.** Procédé selon la revendication 5, dans lequel le peptide employé au stade (i) a été préparé par synthèse en phase solution.

**8.** Procédé pour la préparation d'un composé de la formule (1) selon la revendication 1, dans laquelle Y est du méthylène, ou d'un de ses sels physiologiquement acceptables, lequel procédé consiste à:

(i) synthétiser, soit un peptide qui a une structure de chaîne de la formule:

A-Thr-Ala-Thr-Asu-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-Val-Gly-Ser-Lys-Ala-Phe-$Q_2$

dans laquelle A et $Q_2$ sont comme définis dans la revendication 5, et dans laquelle des groupes de chaîne latérale sont facultativement protégés, soit une partie de ce peptide contenant la séquence:

A-Thr-Ala-Thr-Asu-;

(ii) cycliser la séquence A-Thr-Ala-Thr-Asu pour former ainsi l'unité

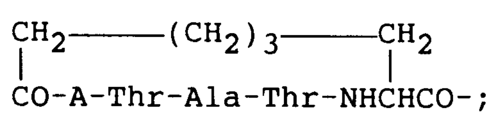

(iii) synthétiser le reste de la séquence du peptide de la formule (1) lorsqu'il n'est pas synthétisé au stade (i);

(iv) enlever tous les groupes protecteurs de groupes fonctionnels de chaîne latérale facultativement présents et, lorsque $Q_2$ n'est pas $-NH_2$, convertir le groupe carboxyle terminal de -Phe- en un groupe amide; et

(v) si on le désire, convertir le composé résultant de la formule (1) en un de ses sels physiologiquement acceptables.

**9.** Procédé pour la préparation d'un composé de la formule (1) comme défini dans la revendication 1, dans laquelle Y est du méthylène, ou d'un de ses sels physiologiquement acceptables, lequel procédé consiste à:

(i) synthétiser une partie du composé de la formule (1) qui a la structure

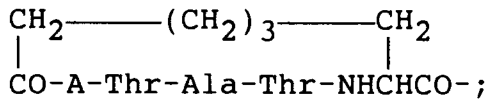

dans laquelle A est comme défini dans la revendication 5, et dans laquelle des groupes fonctionnels de chaîne latérale sont facultativement protégés;

(ii) synthétiser l'autre partie du composé de la formule (I), en procurant facultativement des groupes protecteurs pour des groupes fonctionnels de chaîne latérale;

(iii) condenser les parties synthétisées dans les stades (i) et (ii);

(iv) enlever tous les groupes protecteurs de groupes fonctionnels de chaîne latérale facultativement présents et, si le groupe carboxyle terminal de -Phe- n'a pas été converti en un groupe amide, effectuer cette conversion; et

(v) si on le désire, convertir le composé résultant de la formule (1) en un de ses sels physiologiquement acceptables.

**10.** Composition pharmaceutique comprenant un composé de la formule (1) comme défini dans la revendication 1 ou un de ses sels physiologiquement acceptables, en tant qu'ingrédient actif, et un véhicule ou un diluant pharmaceutiquement acceptable.

**Revendications pour l'Etat contractant suivant : ES**

**1.** Procédé pour la préparation d'un composé de la formule (1):

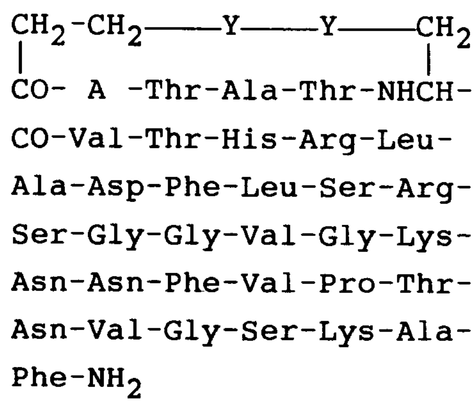

dans laquelle Y est du soufre et A est Asn ou Asp, ou d'un de ses sels physiologiquement acceptables, lequel procédé consiste à:

(i) synthétiser un peptide qui a une structure de chaîne de la formule:

$Q_1$-S-$(CH_2)_2$-CO-A-Thr-Ala-Thr-Cys-Val-Thr-His-Arg-Leu-
Ala-Asp-Phe-Leu-Ser-Arg-Ser-Gly-Gly-Val-Gly-Lys-Asn-Asn-
Phe-Val-Pro-Thr-Asn-Val-Gly-Ser-Lys-Ala-Phe-$Q_2$,

dans laquelle A est Asn ou Asp, $Q_1$ est de l'hydrogène ou un groupe protecteur de mercapto et $Q_2$ est facultatif, mais, s'il est présent, est $-NH_2$, ou, avec le groupe carboxyle en provenance de -Phe-, représente un groupe amide protégé, et dans lequel des groupes fonctionnels de chaîne latérale sont facultativement protégés;

(ii) lorsque Q est un groupe protecteur de mercapto et/ou lorsque le groupe mercapto de chaîne latérale de Cys est protégé, enlever le ou chaque groupe protecteur;

(iii) oxyder les groupes mercapto libres pour former un pont disulfure;

(iv) retirer tous les groupes protecteurs de groupes fonctionnels de chaîne latérale facultativement présents et, lorsque $Q_2$ n'est pas $-NH_2$, convertir le groupe carboxyle terminal de Phe en un groupe amide; et

(v) si on le désire, convertir le composé résultant de la formule (1) en un de ses sels physiologiquement acceptables.

**2.** Procédé selon la revendication 1, dans lequel le peptide employé au stade (i) a été préparé par synthèse en phase solide.

**3.** Procédé selon la revendication 1, dans lequel le peptide employé au stade (i) a été préparé par synthèse en phase solution.

**4.** Procédé pour préparer un composé de la formule (1) selon la revendication 1, dans laquelle Y est du méthylène et A est Asn ou Asp, ou un de ses sels physiologiquement acceptables, lequel procédé consiste à:

(i) synthétiser, soit un peptide qui a une structure de chaîne de la formule:

A-Thr-Ala-Thr-Asu-Val-Thr-His-Arg-Leu-Ala-Asp-Phe-Leu-Ser-Arg-Ser-Gly-Gly-Val-Gly-Lys-Asn-Asn-Phe-Val-Pro-Thr-Asn-val-Gly-Ser-Lys-Ala-Phe-$Q_2$

dans laquelle A et $Q_2$ sont comme définis dans la revendication 1, et dans laquelle des groupes de chaîne latérale sont facultativement protégés, soit une partie de ce peptide contenant la séquence:

A-Thr-Ala-Thr-Asu-;

(ii) cycliser la séquence A-Thr-Ala-Thr-Asu pour former ainsi l'unité

```
CH2           (CH2)3     CH2
|                        |
CO- A -Thr-Ala-Thr-NHCHCO-;
```

(iii) synthétiser le reste de la séquence du peptide de la formule (1) lorsqu'il n'est pas synthétisé au stade (i);

(iv) enlever tous les groupes protecteurs de groupes fonctionnels de chaîne latérale facultativement présents et, lorsque $Q_2$ n'est pas $-NH_2$, convertir le groupe carboxyle terminal de -Phe- en un groupe amide; et

(v) si on le désire, convertir le composé résultant de la formule (1) en un de ses sels physiologiquement acceptables.

**5.** Procédé pour préparer un composé de la formule (1) selon la revendication 1, dans lequel Y est du méthylène et A est Asn ou Asp, ou un de ses sels physiologiquement acceptables, lequel procédé consiste à:

(i) synthétiser une partie du composé de la formule (1) qui a la structure:

```
CH2           (CH2)3     CH2
|                        |
CO- A -Thr-Ala-Thr-NHCHCO-;
```

dans laquelle A est comme défini dans la revendication 1, et dans lequel des groupes fonctionnels de chaîne latérale sont facultativement protégés;

(ii) synthétiser l'autre partie du composé de la formule (1), procurant facultativement les groupes protecteurs pour des groupes fonctionnels de chaîne latérale;

(iii) condenser les parties synthétisées aux stades (i) et (ii);

(iv) enlever tous les groupes protecteurs de groupes fonctionnels de chaîne latérale facultativement présents et, si le groupe carboxyle terminal de -Phe- n'a pas été converti en un groupe amide, effectuer la conversion; et

(v) si on le désire, convertir le composé résultant de la formule (1) en un de ses sels physiologiquement acceptables.

**6.** Procédé selon l'une quelconque des revendications précédentes, qui consiste en outre à formuler le composé obtenu de la formule (1) ou son sel physiologiquement acceptable en une composition pharmaceutique avec un véhicule ou un diluant pharmaceutiquement acceptable.

# FIG. 1

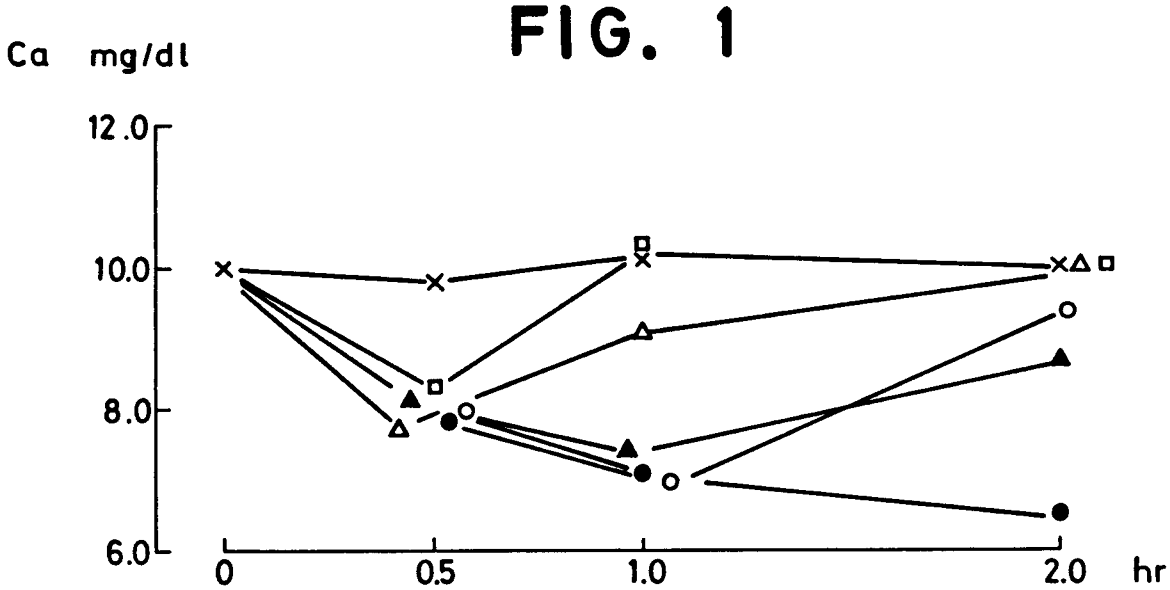


# FIG. 2

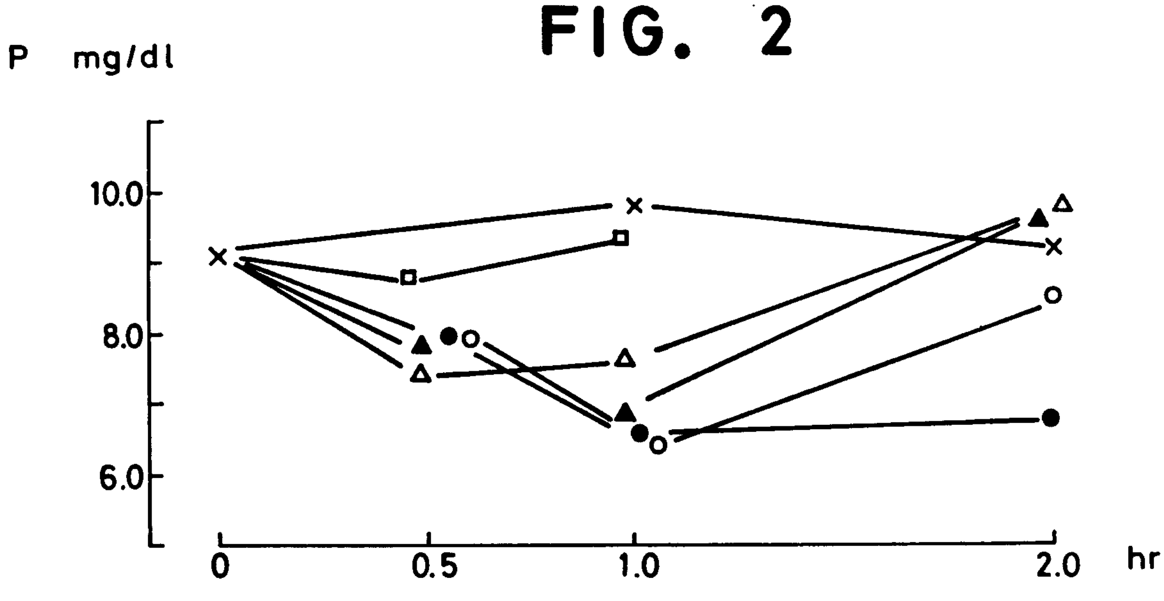

FIG. 3

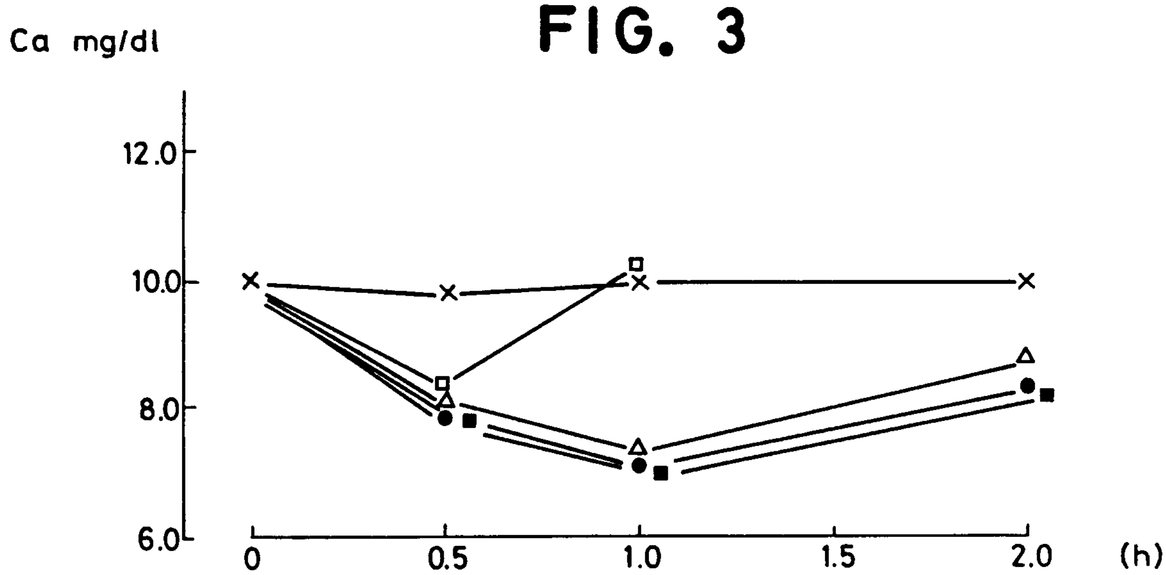


FIG. 4

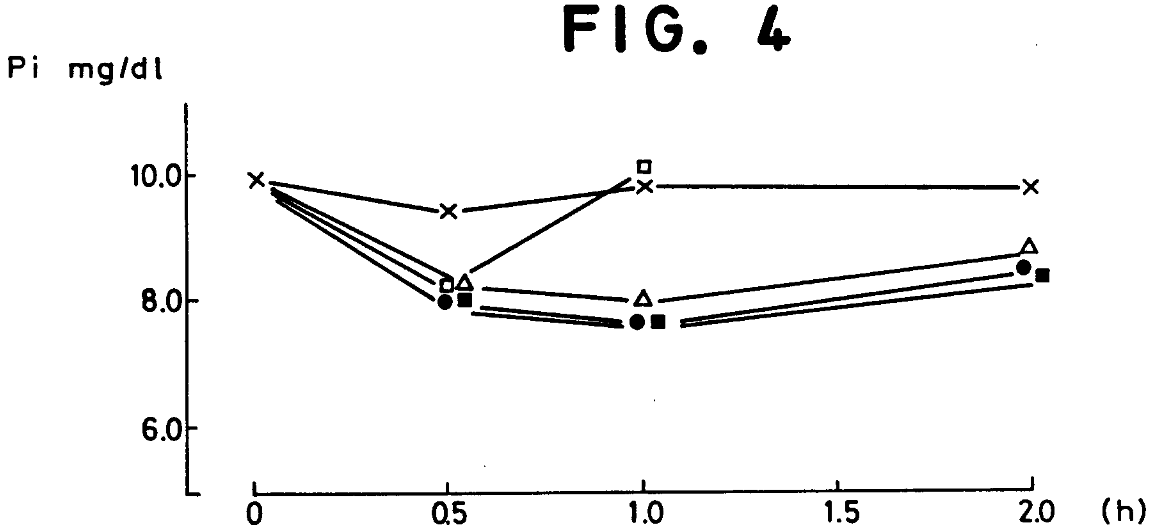

FIG. 5

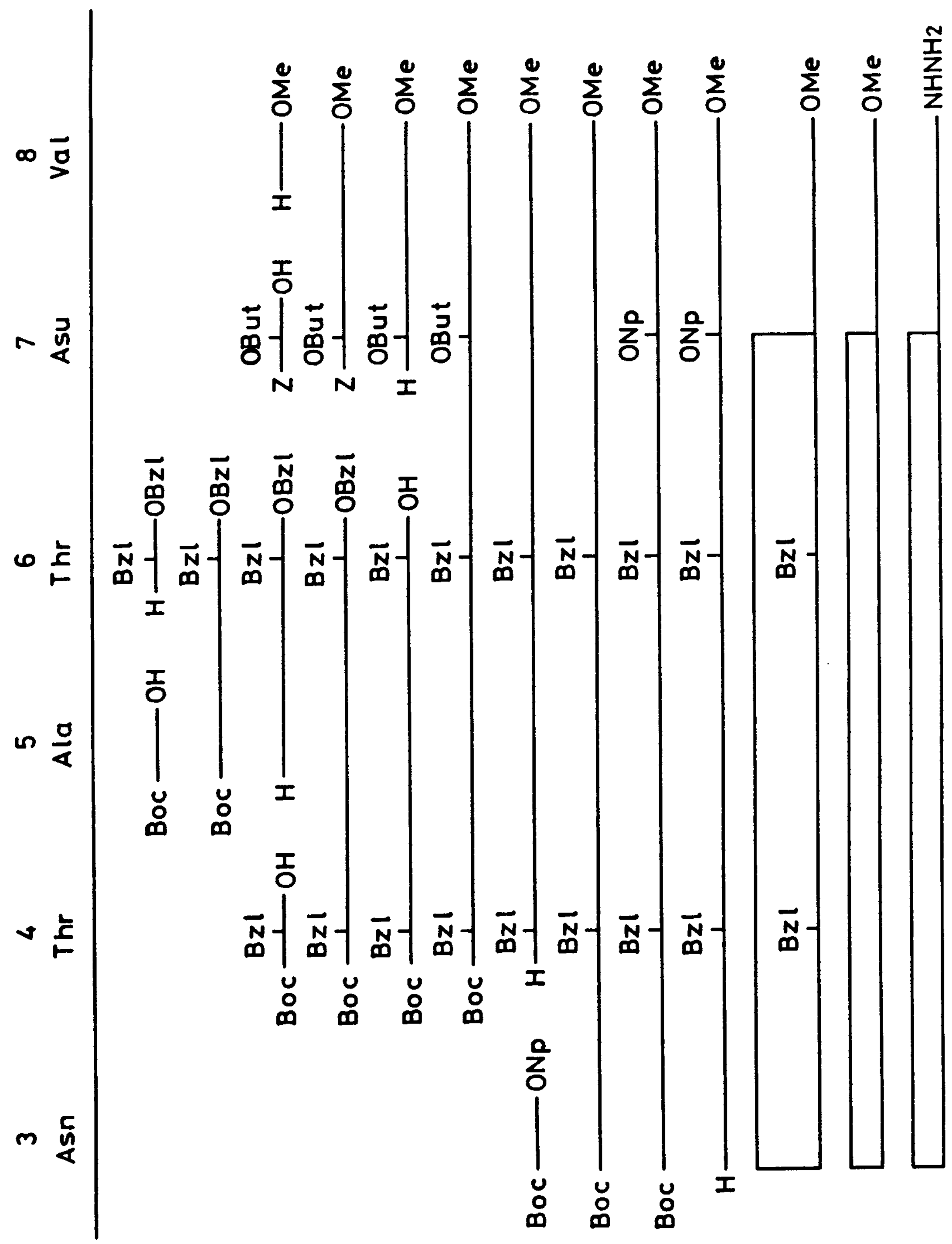

FIG. 6